(19) Europäisches Patentamt
European Patent Office
Office européen des brevets

(11) Veröffentlichungsnummer: **0 503 346 A2**

(12) # EUROPÄISCHE PATENTANMELDUNG

(21) Anmeldenummer: **92103089.6**

(22) Anmeldetag: **24.02.92**

(51) Int. Cl.[5]: **C12N 9/50**, C12N 15/57

(30) Priorität: **01.03.91 DE 4106525**

(43) Veröffentlichungstag der Anmeldung:
**16.09.92 Patentblatt 92/38**

(84) Benannte Vertragsstaaten:
**AT BE CH DE DK FR GB IT LI NL SE**

(71) Anmelder: **Solvay Enzymes GmbH & Co. KG**
**Hans-Böckler-Allee 20, Postfach 220**
**W-3000 Hannover 1(DE)**
Anmelder: **GESELLSCHAFT FüR BIOTECHNOLOGISCHE FORSCHUNG mbH (GBF)**
**Mascheroder Weg 1**
**W-3300 Braunschweig(DE)**

(72) Erfinder: **Vetter, Roman**
**Warneckeweg 1**
**W-3167 Burgdorf(DE)**
Erfinder: **Wilke, Detlef**
**Landwehr 6**
**W-3015 Wennigsen(DE)**
Erfinder: **Amory, Antoine**
**Avenue Bel Air, 44**
**B-1330 Rixensart(BE)**
Erfinder: **Clippe, André**
**Rue Warichet, 12**
**B-1457 Nil-St.-Vincent(BE)**
Erfinder: **Aehle, Wolfgang**
**Bürgerstrasse 18**
**W-3300 Braunschweig(DE)**
Erfinder: **Schomburg, Dietmar**
**Lübeckstrasse 50**
**W-3300 Braunschweig(DE)**
Erfinder: **Sobek, Harald**
**Karlstrasse 34**
**W-3300 Braunschweig(DE)**
Erfinder: **Mücke, Ingo**
**Knickstrasse 63 A**
**W-3013 Barsinghausen(DE)**

(74) Vertreter: **Lauer, Dieter, Dr.**
**c/o Solvay Deutschland GmbH, Postfach 220**
**W-3000 Hannover(DE)**

(54) **Verfahren zur Verbesserung der Stabilität von hochalkalischen Proteasen.**

(57) Beschrieben wird die Stabilisierung hochalkalischer Wasch- und Reinigungsmittel-Proteasen gegen destabilisierende Einwirkung von anionischen Tensiden. Zu ihrer Stabilisierung werden durch mutierte DNA-Sequenzen transformierte Mikroorganismen eingestzt. Die DNA-Sequenzen werden gewonnen, indem man ausgehend von DNA-Sequenzen, die für die hochalkalische Protease codieren, diese DNA-Sequenzen in bestimmten Positionen derart durch gerichtete Mutationen verändert, daß das Codon, in welchem sich die Mutation befindet, nunmehr für eine gegenüber der ursprünglichen Aminosäure andere Aminosäure mit einem voluminöseren und/oder ionischen Aminosäurerest codiert.

EP 0 503 346 A2

Die vorliegende Erfindung bezieht sich auf ein Verfahren zur Verbesserung der Stabilität von hochalkalischen Proteasen gegenüber anionischen Tensiden durch gerichtete Mutagenese von für diese Proteasen codierenden DNA-Sequenzen und nachfolgende Expression dieser Proteasen mit verbesserter anionischer Tensidstabilität.

Hochalkalische Proteasen sind spezielle Enzyme, die gewonnen werden durch Kultivierung von Mikroorganismen, insbesondere durch Kultivierung von Bacillus-Spezies, die wie z.B. Bacillus alcalophilus, die gewünschte hochalkalische Protease produzieren und in das Kulturmedium ausscheiden, aus welchem die Protease isoliert werden kann. Diese hochalkalischen Proteasen unterscheiden sich von gewöhnlichen alkalischen Proteasen, wie sie durch Kultivierung von Bacillus-Spezies, wie insbesondere z.B. B. subtilis, B. amyloliquefaciens und B. licheniformis, gewonnen werden können.

Diese hochalkalischen Proteasen sind wertvolle industrielle Produkte mit vorteilhaften Anwendungen, insbesondere in der Waschmittelindustrie, da sie Protein enthaltende Verunreingungen entfernen. Um wirksam zu sein, müssen diese Proteasen nicht nur proteolytische Aktivität unter Waschbedingungen (pH-Wert, Temperatur) besitzen, sondern müssen darüber hinaus auch mit anderen Waschmittelbestandteilen, insbesondere z.B. in Kombination mit Tensiden, verträglich sein, d.h. in Gegenwart dieser Substanzen ausreichende Stabilität und ausreichende Wirksamkeit aufweisen. Hierbei können insbesondere die in Wasch- und Reinigungsmittelzusammensetzungen häufig verwendeten Tenside anionischen Typs die Stabilität der verwendeten Waschmittelprotease negativ beeinflussen, so daß die Aktivität der Protease in Gegenwart des anionischen Tensids rasch absinkt und die Aktivität selbst bei kurzen Waschzyklen von etwa 30 Minuten kaum ausreichend genutzt werden kann. Die auf die mangelnde Stabilität der Protease gegenüber anionischen Tensiden zurückzuführende unzureichende Ausnutzung der proteolytischen Aktivität während des Wasch- bzw. Reinigungsvorganges bedingt somit auch deutlich verminderte Wasch- bzw. Reinigungsleistungen. Eine gute Stabilität der Wasch- und Reinigungsmittelprotease gegenüber anionischen Tensiden ist insbesondere für Flüssigformulierungen von Wasch- und Reinigungsmitteln erforderlich, da die Proteasen in diesen Formulierungen nicht wie in Pulverformulierungen durch Beschichtungsverfahren gegen destabilisierende Einwirkungen anderer Formulierungskomponenten, wie insbesondere anionische Tenside, geschützt werden können.

Es bestand daher die Aufgabe, die Stabilität von hochalkalischen Proteasen gegen destabilisierende Einwirkung von anionischen Tensiden zu verbessern und ein hierfür geeignetes Verfahren anzugeben.

Die Aufgabe wird gelöst durch das erfindungsgmäße Verfahren zur Verbesserung der Stabilität von hochalkalischen Proteasen gegen destabilisierende Einwirkung von anionischen Tensiden, welches sich dadurch auszeichnet, daß man in einer Protease mit einer Aminosäurensequenz mit mindestens 80 % Homologie zu der in Fig. 1 angegebenen Aminosäurensequenz in einer der Positionen 42, 57, 96, 114, 115, 166, 255 der Fig. 1 oder in einer der dazu homologen Positionen die in der betreffenden Position befindliche Aminosäure durch eine Aminosäure mit einem voluminöseren und/oder ionischen Aminosäurerest austauscht. In einer bevorzugten Ausgestaltung der Erfindung werden solche hochalkalischen Proteasen eingesetzt, deren Aminosäurensequenzen eine Homologie von mindestens 90 %, insbesondere aber mindestens 95 %, zu der Aminosäurensequenz der Fig. 1 besitzen.

Unter Homologie zu der in Fig. 1 angebenen Aminosäurensequenz wird hier die strukturelle Verwandtschaft der betreffenden Aminosäurensequenzen zu der in Fig. 1 angebenen Aminosäurensequenz verstanden. Zur Bestimmung der Homologie werden jeweils die einander strukturell entsprechenden Abschnitte der Aminosäurensequenz der Fig. 1 und der damit zu vergleichenden Aminosäurensequenz so zur Deckung miteinander gebracht, daß maximale Strukturübereinstimmung zwischen den Aminosäurensequenzen besteht, wobei durch Deletion oder Insertion einzelner Aminosäuren verursachte Unterschiede berücksichtigt und durch entsprechende Verschiebungen von Sequenzabschnitten ausgeglichen werden. Die Zahl der nunmehr in den Sequenzen miteinander übereinstimmenden Aminosäuren ("homologe Positionen") bezogen auf die Gesamtzahl der in der Sequenz der Fig. 1 enthaltenen Aminosäuren gibt dabei die Homologie in % an. Abweichungen in den Sequenzen können sowohl durch Variation, Insertion als auch Deletion von Aminosäuren bedingt sein.

Es versteht sich dementsprechend von selbst, daß bei Einsatz von zur Figur 1 wenigstens zu 80 % homologen hochalkalischen Proteasen, die mit Bezug auf die Fig. 1 bezeichneten Aminosäurenpositionen sich auf die dazu homologen Positionen der jeweils eingesetzten Protease beziehen. Deletionen oder Insertionen in den Aminosäurensequenzen der zu Fig. 1 homologen Proteasen können zu einer relativen Verschiebung der Aminosäurenpositionen führen, so daß in homologen Teilstücken von zueinander homologen Aminosäurensequenzen die numerischen Bezeichnungen der einander entsprechenden Aminosäurepositonen nicht identisch zu sein brauchen.

Für den Austausch der in einer der Austauschpositionen ursprünglich befindlichen Aminosäuren sind zum einen solche Aminosäuren geeignet, die gegenüber der ursprünglichen Aminosäure einerseits einen neutralen aber zumindest voluminöseren oder andererseits entgegen der ursprünglichen Aminosäure einen zumindest ionischen Aminosäurerest aufweisen. Zum anderen sind für den Austausch der ursprünglichen Aminosäure aber auch solche geeignet, die gegenüber der ursprünglichen Aminosäure einen voluminöseren und zudem ionischen Aminosäurerest besitzen. Die ionischen Aminosäurereste können hierbei sowohl kationisch als auch anionisch sein. Als Beispiel für Aminosäuren, die zum Austausch der in den Austauschpositionen ursprünglich befindlichen Aminosäuren geeignet sein können, seien insbesondere die Aminosäuren aus der Gruppe Arginin, Lysin, Glutaminsäure, Glutamin, Asparaginsäure und gewünschtenfalls auch solche Aminosäuren aus der Gruppe Methionin, Phenylalanin, Tyrosin und Tryptophan genannt. Hierbei können diese Aminosäuren bspw. wie folgt in Untergruppen eingeteilt werden: Glutamin, Methionin, Phenylalanin, Tyrosin und Tryptophan als Aminosäuren mit voluminöseren neutralen Aminosäureresten; Asparaginsäure und Glutaminsäure als Aminosäuren mit anionischen Aminosäureresten, wobei der Glutaminsäurerest zudem auch voluminöser ist; Lysin und Arginin als Aminosäuren mit kationischem Aminosäurerest, wobei deren Aminosäurereste zudem auch voluminöser ist. Zweckmäßige Beispiele für erfindungsgemäß stabilisierte hochalkalische Proteasen sind demgemäß bspw. solche Proteasen der oben angegebenen Art bei denen die ursprüngliche Aminosäure wie folgt durch eine andere Aminosäure ausgetauscht ist: in Position 42, 57, 96, 114, 115, 166 oder 255 durch Lysin oder Arginin, in Position 42 oder 255 durch Glutamin oder Glutaminsäure; in Position 42 durch Methionin oder in Position 255 durch Asparaginsäure. Als weitere Beispiele für einen erfindungsgemäßen Austausch von Aminosäuren in den Proteasen sind zu nennen, der Austausch der ursprünglichen Aminosäure in Position: 42 oder 57 durch Tyrosin; 42 durch Phenylalanin; 57 durch Tryptophan.

Eine sehr gute Stabilisierung von hochalkalischen Proteasen gegen destabilisierende Einwirkungen von anionischen Tensiden wird nach dem erfindungsgemäßen Verfahren insbesondere z.B. dann erzielt, wenn man in einer der Proteasen mit einer Aminosäurensequenz mit mindestens 80 %, vorzugsweise mindestens 90 %, insbesondere aber mindestens 95 % Homologie zu der in Fig. 1 angegebenen Aminosäurensequenz in einer der Positionen 42, 57, 114, 255 der Fig. 1 oder in einer der dazu homologen Positionen die in der betreffenden Position befindliche Aminosäure z.B. durch die Aminosäure Arginin austauscht. So weisen die erfindungsgemäß durch Aminosäurenaustausch in einer der vorstehenden bevorzugten Positionen stabilisierten hochalkalischen Proteasen nach 30-minütigem Streßtest in Gegenwart von repräsentativen anionischen Tensiden eine um die Selbstverdauung korrigierte Restaktivität auf, die wenigstens etwa das zwei- bis vierfache der entsprechenden Restaktivität der jeweils zugrundeliegenden unstabilisierten Protease beträgt. Ganz besonders gut ist z.B. die Stabilisierung durch Aminosäurenaustausch gegen Arginin in den Positionen 42, 57 der Fig. 1 oder dazu homologen Positionen; in derart stabilisierten Proteasen kann nach 30-minütigen Streßtest in Gegenwart von repräsentativen anionischen Tensiden die Restaktivität sogar etwa das vier- bis siebenfache der entsprechenden Restaktivität der zugrundeliegenden unstabilisierten Protease betragen.

Die durch das erfindungsgemäße Verfahren stabilisierbaren hochalkalischen Proteasen sind bspw. solche, die durch Kultivierung von Mikroorganismen wie z.B. Bacillus alcalophilus erhältlich sind. Stellvertretend für die Gruppe solcher hochalkalischer Bacillus-Proteasen wird das erfindungsgemäße Verfahren zur Stabilisierung dieser Proteasen gegen anionische Tenside hier insbesondere an solchen hochalkalischen Proteasen näher beschrieben, die durch Kultivierung von Bacillus-Spezies erhältlich sind, die die Identifizierungsmerkmale von Bacillus alcalophilus DSM 5466 aufweisen. Die erfindungsgemäß stabilisierten Proteasen besitzen somit eine durch die oben angegebene Homologie definierte Verwandtschaft zu der aus Bacillus alcalophilus DSM 5466 erhältlichen Protease, deren Aminosäurensequenz in Fig. 1 als Bezugspunkt stellvertretend für die erfindungsgemäß stabilisierbaren Proteasen angegeben ist. Diese hochalkalischen Proteasen besitzen Molekulargewichte von 26000 bis 28000 g/mol, gemessen durch SDS-Polyacrylamid-Gelelektrophorese gegenüber Referenzproteinen mit bekanntem Molekulargewicht. Sie zeichnen sich weiterhin durch ein pH-Optimum im Bereich von 10 bis 12,5 aus, wobei unter pH-Optimum derjenige pH-Bereich verstanden wird, in dem die Proteasen maximale proteolytische Aktivität aufweisen und die Proteasen besitzen eine gute pH-Stabilität.

Bei der Ausführung des erfindungsgemäßen Verfahrens werden die in der Aminosäurensequenz der jeweils zu stabilierenden Protease auszutauschenden Aminosäuren durch gerichtete Mutagenese der für die Aminosäurensequenz dieser Protease codierenden DNA-Sequenz und nachfolgende Expression der mutierten DNA-Sequenz mit Hilfe eines geeigneten Mikroorganismus durch die in die Aminosäurensequenz erfindungsgemäß einzubringende Aminosäure mit voluminöserem und/oder ionischem Aminosäure ausgetauscht. Hierbei kann im Einzelnen so vorgegangen werden, daß man

a) zunächst aus einem geeigneten Bakterium, welches eine hochalkalische Protease mit einer Aminosäurensequenz mit mindestens 80 %, vorzugsweise mindestens 90 %, insbesondere aber mindestens 95 % Homologie zu der Aminosäurensequenz der Fig. 1 produziert, die für die Protease codierende DNA-Sequenz (d.h. das Strukturgen der Protease) isoliert,

b) die Nukleotidabfolge dieser DNA-Sequenz bestimmt,

c) in der nunmehr bekannten DNA-Sequenz solche Mutationen erzeugt, daß die mutierte DNA-Sequenz nun für eine hochalkalische Protease, in der eine Aminosäure der Ursprungsprotease in einer der vorstehend gegebenen Positionen durch eine Aminosäure mit einem voluminöserem und/oder eine ionischen Aminosäurerest ausgetauscht ist, codiert,

d) nachfolgend die mutierte DNA-Sequenz in einen geeigneten Expressionsvektor einbaut und

e) mit dem erhaltenen Expressionsvektor einen geeigneten Mikroorganismus, welcher schließlich zur Herstellung der mutierten hochalkalischen Protease eingesetzt werden kann, transformiert.

Die Verfahrensschritte zur erfindungsgemäßen Stabilisierung und zur Gewinnung der erfindungsgemäß stabilisierten hochalkalischen Proteasen, sowie die hierbei erhaltenen Produkte sowie Zwischenprodukte in Form von DNA-Sequenzen, Vektoren, insbesondere Expressionsvektoren, und transformierten Mikroorganismen werden nachfolgend näher beschrieben.

Die Strukturgene, die für Aminosäurensequenzen hochalkalischer Proteasen mit wenigstens 80 % Homologie zu der in Fig. 1 angegebenen Aminosäurensequenz codieren, können nach an sich bekannten, allgemeinen Methoden erhalten werden. Hierzu wird z.B. aus einem Bakterium ("Donor-Bakterium"), insbesondere aus einer Bacillus-Spezies, die die hochalkalische Protease produziert, die chromosomale DNA nach an sich bekannten Methoden isoliert und mit geeigneten Restriktionsendonukleasen partiell hydrolysiert. Restriktionsendonukleasen sind Enzyme, die substratsspezifisch doppelsträngige DNA dadurch in Fragmente zerlegen, daß sie Phosphatdiesterbindungen zwischen einzelnen Nukleotidbausteinen der DNA spalten. Alle Restriktionsendonukleasen vermögen bestimmte Basensequenzen der DNA zu erkennen, welche für die Aktivität der betreffenden Restriktionsendonukleasen spezifische Wirkungsorte (Schnittstellen) markieren. Beim Schneiden (Restriktion) doppelsträngiger DNA entstehen bei einigen Restriktionsendonukleasen spezifische, sogenannte "überstehende Enden", die unter bestimmten Renaturierungsbedingungen wieder miteinander oder mit entsprechenden (komplementären) überstehenden Enden anderweitig gewonnener DNA-Fragmente verbunden (ligiert) werden können (Rekombination). Beim Schneiden mit anderen Restriktionsendonukleasen entstehen DNA-Doppelstränge mit glatten Enden. Diese DNA-Doppelstränge mit glatten Enden können mit beliebigen DNA-Doppelsträngen, die ebenfalls glatte Enden besitzen,rekombiniert werden.

Die erhaltenen Restriktionsfragmente der Donor-DNA können z.B. durch Gelelektrophorese nach Größe aufgetrennt und die Fragmente gewünschter Größe dann mit einer geeigneten, doppelsträngigen Vektor-DNA rekombiniert werden.

Vektoren sind DNA-Moleküle, die sich als Transportmoleküle (Vehikel) zur Einschleusung (Transformation) von Fremd-DNA in Wirtszellen eignen, dort ggf. autonom replizierbar sind und gegebenenfalls noch sogenannte Marker besitzen. Marker sind DNA-Fragmente, die für bestimmte, beobachtbare Eigenschaften (z.B. Antibiotika-Resistenz) codieren und der nachfolgenden Selektion der transformierten Mikroorganismen (Transformanten) dienen. Häufig verwendete Vektoren sind die sogenannten Plasmide, d.h. extrachromosomale, ringförmige, doppelsträngige Bakterien-DNA, die sich durch geeignete Methoden in andere Mikroorganismen einbringen läßt und dort vermehrbar ist.

Mit der in vitro rekombinierten DNA (Vektor + Restriktionsfragmente der Donor-DNA) können Bakterien, vorzugsweise eine Bacillus-Spezies, transformiert werden und die Transformanten nach der bekannten Markereigenschaft (z.B. Neomycin-Resistenz) selektiert werden. Man erhält so Klone, d.h. genetisch identische Transformanten. Unter diesen Transformanten können solche, die vermehrt Protease ausscheiden, auf proteinhaltigen Platten gesucht und danach isoliert werden. Aus einem Klon mit Proteaseaktivität wird schließlich die in diesen Transformanten eingeführte Plasmid-DNA isoliert und durch erneute Transformation eines Bakteriums überprüft, ob die Proteaseaktivität Plasmid-gebunden ist, d.h. ob die Proteaseaktivität mit der Markereigenschaft gekoppelt ist.

Das so isolierte Plasmid enthält neben der Vektor-DNA mit bekannten Restriktionsstellen das gewünschte Strukturgen für die zu stabilisierende hochalkalische Ausgansprotease und weitere, hier aber nicht benötigte DNA-Sequenzen aus dem Donor-Bakterium. Um den Aufwand für die nachfolgende Sequenzierung des Strukturgens der zu stabilisierenden hochalkalischen Protease möglichst gering zu halten, empfiehlt es sich, vor der eigentlichen Sequenzierung die zusätzlichen, nicht benötigten DNA-Sequenzen aus der Donor-DNA-Sequenz zu eleminieren und die Donor-DNA-Sequenz im wesentlichen auf das Strukturgen für die Protease zu reduzieren. Hierzu wird z.B. das Plasmid, welches das Strukturgen und die zusätzliche DNA-Sequenz umfaßt, mit einer Anzahl verschiedener Restriktionsendonukleasen geschnitten (restringiert), die erhaltenen DNA-Fragmente durch Gelelektrophorese nach Größe getrennt und anhand des gefundenen Bandenmusters eine Restriktionskarte erstellt. Es werden so die Restriktionsstellen, die im Bereich der Donor-DNA-Sequenz angesiedelt sind, ermittelt. Die Kenntnis der Restriktionskarte des Plasmids ermöglicht es nunmehr, aus diesem durch Schneiden mit ausgewählten Restriktionsendonukleasen ein DNA-Fragment aus der Donor-DNA-Sequenz herauszuschneiden, welches im wesentlichen nur noch das Strukturgen für die hochalkalische Protease, die zugehörigen Pre- und Pro-Einheiten, sowie die für die Genexpression benötigte Promotor-Einheit umfaßt.

Durch den Wiedereinbau dieser in der Größe reduzierten Donor-DNA-Sequenz in einen geeigneten Vektor kann ein neuer, replizierbarer Vektor erhalten werden, dessen Fähigkeit zur Expression der hochalkalischen Ausgangsprotease überprüft werden kann, indem man ein Bakterium, insbesondere eine Bacillus-Spezies, mit diesem Vektor transformiert, den erhaltenen Transformanten kultiviert und auf Proteaseaktivität überprüft. Ein Beispiel für einen solchen reduzierten Vektor mit der Bezeichnung pCLEAN4 gibt die Restriktionskarte der Fig. 2 wieder.

Zur Bestimmung der Nukleotidsequenz (Sequenzierung) des Proteasestrukturgens wird zunächst der vorstehend beschriebene Vektor in einem geeigneten Mikroorganismus repliziert, und das Proteasegen isoliert. Dieses wird sodann in einen Phagemiden subkloniert und die erhaltenen Phagemiden anschließend in einen geeigneten Mikroorganismus z.B. E. coli transformiert und durch Kultivierung der Transformanten einzelsträngige, das Proteasegen enthaltende DNA produziert. Die gebildete einzelsträngige DNA wird isoliert und der Sequenzierung zugeführt. Die Sequenzierung wird nach an sich bekannten Methoden ausgeführt, indem man z.B. die Einzelstrang-DNA mit dem Proteasegen nach Maxam und Gilbert einer basenspezifischen partiellen chemischen Spaltung zuführt (1980, in Methods in Enzymology, Grossmann L., Moldave K., eds., Academic Press Inc., New York und London, Vol. 65, 499), oder indem man z.B. die Einzelstrang-DNA mit dem Proteasegen als Matritze für die partielle Synthese von Teilstücken des komplementären DNA-Stranges nach der Dideoxy-Kettenterminator-Methode nach Sanger et al. (1977, Proc. Natl. Acad. Sci. USA 74: 5463) einsetzt.

Die ermittelte Nukleotidsequenz kann nunmehr mit Hilfe des genetischen Codes (ein Triplett-Wort = Codon steht für eine definierte Aminosäure) in die Aminosäurensequenz der Protease übersetzt werden. Zur Bestimmung des Anfangspunktes der Aminosäurensequenz des reifen Protease-Enzyms (d.h. das Enzym ohne die Pre- und Pro-Einheiten) wird am N-terminalen Ende der reifen Protease ein kurzes Stück der Aminosäurenabfolge durch an sich bekannte Methoden zur Bestimmung von Aminosäurensequenzen in Peptiden bestimmt. Die bekannte N-terminale Aminosäurensequenz kann nun anhand des genetischen Codes dem entsprechenden Teilstück der obigen Nukleotidsequenz zugeordnet werden und so der Anfangspunkt der für die reife Protease codierenden DNA-Sequenz festgelegt werden. Die weitere Aminosäurenabfolge der Protease ergibt sich dann zwangsläufig aus der DNA-Sequenz durch Zuordnung der nachfolgenden Aminosäuren mit Hilfe des genetischen Codes.

Erfindungsgemäß wird die für die hochalkalische Protease codierende DNA-Sequenz durch Änderung der entsprechenden Codons derart mutiert, daß die mutierte DNA-Sequenz für eine gegen Einwirkung von anionischen Tensiden stabilisierte hochalkalische Protease codiert, in deren Aminosäurensequenz in einer der Positionen 42, 57, 96, 114, 115, 166, 255, vorzugsweise 42, 57, 114, 255 bzw. insbesondere 42, 57 der Fig. 1 oder in einer der dazu homologen Positionen die betreffende Aminosäure durch eine Aminosäure mit einem voluminöseren und/oder ioninschen Aminosäurerest ausgetauscht ist.

Die Einführung der Mutationen in die für die hochalkalische Protease codierende DNA wird durch an sich bekannte Methoden zur gerichteten Mutagenese bewerkstelligt. Hierzu wird aus geeigneten Vektoren (Phagemide), z.B. aus pCLMUTN1 der Fig. 4 oder pCLMUTC1 der Fig. 5, gegebenenfalls unter Mithilfe eines Helfer-Phagen, ringförmige Einzelstrang-DNA erzeugt, die das gesamte Strukturgen, oder aber vorzugsweise nur denjenigen Teil (z.B. nur den N-terminalen Teil bzw. den C-terminalen Teil) des Strukturgens der Ursprungsprotease, in welchem die Mutation vorgenommen werden soll, enthält. Mit dieser ringförmigen Einzelstrang-DNA hybridisiert man ein synthetisches, hybridisierfähiges Oligonukleotid, welches in der gewünschten Mutationsstelle ein Basentriplett (Codon) enthält, welches für eine gegenüber der originären Aminosäure in dieser Position erfindungsgemäß auszutauschende Aminosäure, z.B. für Arginin oder Lysin, codiert. Zusätzlich ist das Oligonukleotid gegenüber der zu hybridisierenden, originären Nukleotidsequenz noch derart durch einen oder einige wenige weitere Nukleotidbausteine abgewandelt, daß die Codierung der originären Aminosäurensequenz zwar im Rahmen der Degeneration des genetischen Codes erhalten bleibt, jedoch in der originären Protease-Nukleotidsequenz eine gegebenenfalls vorhandene Restriktionsstelle im synthetischen Oligonukleotid entfernt bzw. eine weitere Restriktionsstelle in das synthetische Oligonukleotid eingeführt wird. Die entfernte bzw. eingeführte Restriktionsstelle dient später zur Identifizierung der Mutanten-DNA-Sequenz gegenüber der Ausgangstyp-DNA-Sequenz mit Hilfe geeigneter Restriktionsendonukleasen. Die Ergänzung der durch Hybridisierung erhaltenen partiell doppelsträngigen DNA-Sequenz zum vollständigen Doppelstrang wird dann durch Zugabe der benötigten Nukleotide und unter Einwirkung von DNA-Polymerase und DNA-Ligase durchgeführt. Die erzeugte ringförmige, doppelsträngige DNA-Sequenz wird nachfolgend als Vektor in einen geeigneten Mikroorganismus transformiert und nach ausreichender Replikation die mutierten DNA-Sequenzen über die unitären Restriktionsendonukleasen-Erkennungsstellen identifiziert und anschließend isoliert.

In einer Variante wird im Verfahren der gerichteten Mutagenese uracylierte Einzelstrang-DNA als Matrize erzeugt und für die Hybridisierung mit den synthetischen Oligonukleotiden verwendet. Nach Beendigung der Reaktionen des Verfahrens der gerichteten Mutagenese kann der Uracilhaltige DNA-Einzelstrang, der als Matrize zur Erzeugung mutierter DNA-Stränge (Vektoren) diente, durch Behandlung mit Uracil-N-Glucosylase beseitigt werden, ohne daß es einer phänotypischen Selektion von Mutanten bedarf. Die Glucosylase-Behandlung kann z.B. mit Hilfe eines geeigneten Mikroorganismus mit Uracil-N-Glucosylase-Aktivität durchgeführt werden, der mit mutierter Vektor-DNA transformiert wurde. Die Replikation kann z.B. in einem E. coli-Stamm vorgenommen werden, der nur den mutierten, nicht-uracylierten DNA-Strang des im Mutationsverfahren erzeugten Doppelstrang-Vektors vermehrt. Hierdurch wird die Selektion der mutierten DNA-Vektoren zusätzlich erleichtert.

Die für die gerichtete Mutagenese benötigten synthetischen Oligonukleotide werden nach an sich bekannten Methoden hergestellt. Beispielsweise kann die Herstellung der Oligonukleotide nach Beaucage S.L. und Caruthers M.H. (1981, Tetrahedron Letters 22: 1859 - 1862) mit $\beta$-Cyanoethyl-phosphoramidit in einem Cyclone-Synthetiser (Biosearch) erfolgen. Die erhaltenen Oligonukleotide können z.B. durch Elution aus Polyacrylamid-Gelen und gegebenenfalls anschließende Entsalzung mit Hilfe von Sephadex-Säulen gereinigt und der weiteren Verwendung zugeführt werden. Die synthetischen Oligonukleotide können direkt als Primer für die DNA-Polymerase im vorstehend beschriebenen Mutagenese-Verfahren dienen. Die synthetischen Oligonukleotidsequenzen umfassen z.B. 20 bis 30 Nukleotidbausteine, die für etwa 7 bis 10 Aminosäuren codieren. Es ist natürlich auch möglich längere Nukleotidsequenzen für die obige Hybridisierung einzusetzen, doch führt dies zu keinen weiteren Vorteilen, solange eine ausreichende Hybridisierungsfähigkeit der kurzkettigen synthetischen Oligonukleotide sichergestellt ist.

Die durch das oben beschriebene Verfahren der gerichteten Mutagenese erhaltenen ringförmigen, doppelsträngigen DNA-Sequenzen mit den eingeführten Mutationen stellen mutierte Vektoren dar, aus denen durch Behandlung mit geeigneten Restriktionsendonukleasen, je nach Fall, das gesamte mutierte Protease-Strukturgen oder das mutierte Teilstück des Protease-Strukturgens herausgeschnitten und in einen geeigneten Expressionsvektor eingebracht (subkloniert) werden kann. Mit diesem Expressionsvektor werden dann geeignete Mikroorganismen, z.B. Bacillus-Spezies, transformiert, die nachfolgend zur Expression und Gewinnung der mutierten hochalkalischen Proteasen unter geeigneten Bedingungen kultiviert werden.

In einer bevorzugten Ausgestaltung der Erfindung wird nicht das gesamte Strukturgen für die gerichtete Mutagenese eingesetzt, sondern nur ein Teilstück desselben, in dem die Mutation erzeugt werden soll. Hierzu wird aus dem Vektor der zur Replikation der Strukturgene dient, z.B. die N-terminale oder C-terminale Hälfte des Strukturgens mit geeigneten Restriktionsendonukleasen herausgeschnitten und in einen passenden Phagemiden subkloniert. Man erhält so Vektoren, die entweder die N-terminale oder die C-terminale Hälfte des Strukturgens der Protease enthalten und die in einem geeigneten Mikroorganismus, z.B. E. coli, zunächst ausreichend repliziert und dann der oben beschriebenen, gerichteten Mutagenese zugeführt werden. Die Mutagenese von Teilstücken des Strukturgenes hat den Vorteil, daß kürzere Einzelstrang-DNA-Sequenzen verwendet werden können und somit nach dem Hybridisierungsschritt mit synthetischen Oligonukleotiden im partiellen DNA-Doppelstrang wesentlich weniger Nukleotide als bei Verwendung der gesamten DNA-Sequenz zu ergänzen sind. Hier durch wird der synthetische Aufwand und zusätzlich die Gefahr unerwünschter zufälliger Mutationen reduziert.

Die mutierten DNA-Sequenzen können aus dem zur Erzeugung der Mutationen dienenden Klonierungsvektoren durch geeignete Restriktionsendonukleasen herausgeschnitten und in entsprechende Restriktionsstellen besitzende Vektoren eingebaut werden, die Vorläufer der eigentlichen, für die Expression der hochalkalischen Protease benötigten Expressionsvektoren darstellen. Diese Vektoren sind so aufgebaut, daß sie außer den geeigneten Restriktionsstellen (z.B. aus einem synthetischen Linker) auch bereits die für die Proteaseexpression in einem Wirtsorganismus benötigten regulatorischen Sequenzen, Signalsequenzen, Promotorsequenzen und die für die Pre- und Proeinheiten der Protease codierenden DNA-Sequenzen enthalten.

Durch die Subklonierung einer mutierten DNA-Sequenz in einen solchen Vektor wird der eigentliche Expressionsvektor für eine optimierte hochalkalische Protease erhalten. Der Einbau der mutierten DNA-Sequenz in diesen Vorläufer des Expressionsvektors erfolgt so, daß ein Expressionsvektor mit geeignetem Leseraster entsteht. Hierbei können mutierte Teilstücke der für die Protease codierenden DNA-Sequenz, z.B. ein C-terminales oder ein N-terminales Teilstück, in bereits das jeweils restliche nicht mutierte Teilstück enthaltende Vektoren eingebaut werden; oder es wird die gesamte für die Protease codierende mutierte DNA-Sequenz in Vektoren, welche noch keine Teilstücke dieser Protease-DNA-Sequenz enthalten, eingebaut. Beispiele für solche, bereits ein Teilstück der nicht-mutierten DNA-Sequenz enthaltende Vorläufervektoren eines Expressionsvektors sind die weiter unten und in den Beispielen näher beschriebenen Vektoren mit der Bezeichnung pAL1N und pAL1C. Ein Vektor, der noch kein Teilstück der Protease-DNA-Sequenz enthält, ist der Vektor pAL1P mit der in Fig. 7 angegebenen Restriktionskarte.

Die Expressionsvektoren-Vorläufer für die bevorzugte Variante der Erfindung (Mutation in der N-terminalen Hälfte oder in der C-terminalen Hälfte) werden z.B. wie folgt erhalten. Zunächst führt man in ein Bacillus-Plasmid eine Polyklonierungsstelle ein. Das so erhaltene Plasmid wird restringiert und mit einem E. coli-Plasmidfragment, welches Marker und für die Replikation wichtige Sequenzteile enthält, rekombiniert. Anschließend werden gegebenenfalls solche Restriktionsstellen z.B. durch gerichtete Mutagenese entfernt, die spätere Verfahrensschritte stören würden. Aus dem so erhaltenen Plasmid wird ein neuer Vektor konstruiert, der die aus dem Bacillus-Plasmid und dem E. coli-Plasmid zur Replikation dienenden DNA-Sequenzen, DNA-Sequenzen für den Promotor, DNA-Sequenzen, die für die Pre-Prosequenz der Protease codieren (erhalten aus z.B. dem Plasmid pCLEAN4 der Fig. 2), und einen synthetischen Linker enthält. Ein Beispiel für ein solches Plasmid mit der Bezeichnung pAL1P gibt die Restriktionskarte der Fig. 7 wieder. Der synthetische Linker ist dabei derart ausgewählt, daß nach Schneiden mit geeigneten Restriktionsendonukleasen entweder mit dem gesamten Ursprungsstrukturgen bzw. dem gesamten mutierten Strukturgen bzw. mit mutierten oder nicht mutierten Teilstücken des Strukturgens rekombiniert werden kann. Zur Herstellung eines Expressionsvektor-Vorläufers, der z.B. mit einer mutierten N-terminalen Hälfte des Strukturgens rekombiniert werden soll, wird in den vorstehend konstruierten Vektor, der die genannten Bacillus-, E. coli-, die Promotor- und die Pre- und Prosequenzen der Protease sowie den synthetischen Linker enthält, durch Schneiden des synthetischen Linkers z.B. zunächst die nicht-mutierte C-terminale Hälfte des Strukturgens der Protease eingeführt. Man erhält so die bereits genannten Vektoren vom Typ pAL1C. Anschließend wird durch nochmaliges Schneiden des synthetischen Linkers die noch fehlende, mutierte N-terminale Hälfte des Protease-Strukturgens eingeführt. Auf diese Weise erhält man einen Vektor vom Typ pAL1NC der Fig. 8. Analoges gilt für den umgekehrten Fall. Es wird dann zunächst die nicht-mutierte N-terminale Hälfte in einen Vektor vom Typ pAL1P und in den so erhaltenen Vektor vom Typ pAL1N später die mutierte C-terminale Hälfte eingeführt, wobei man ebenfalls einen Vektor vom Typ pAL1NC der Fig. 8 erhält.

Mit den oben beschriebenen Expressionsvektoren werden geeignete Bakterien, vorzugsweise Bacillus-Spezies, insbesondere Bacillus subtilis, licheniformis und alcalophilus, transformiert. Die Transformanten werden anschließend in an sich bekannter Weise kultiviert und die gebildete, erfindungsgemäß stabilisierte hochalkalische Protease aus dem Kulturmedium isoliert. Die Expressionsvektoren können hierzu sowohl in Bakterien, die noch zur Bildung von Eigenprotease befähigt sind, als auch in Protease-defiziente Bakterien (die keine Eigenprotease mehr bilden) transformiert werden. Bei Wirtsorganismen mit Eigenproteasebildung kann die erfindungsgemäß stabilisierte hochalkalische Protease gewünschtenfalls durch anschließende Reinigungsoperationen, z.B. durch hochauflösende Flüssigchromatographie (HPLC), von den gebildeten Eigenproteasen befreit werden. Ein solcher Reinigungschritt kann demgegenüber bei Protease-defizienten Wirtsorganismen entfallen, da diese nur (oder im wesentlichen nur) die stabilisierte Protease zu bilden vermögen.

Durch das erfindungsgemäße Verfahren zur Stabilisierung der weiter oben angegebenen hochalkalischen Proteasen gegen destabilierende Einwirkungen von anionischen Tensiden werden auch neue Proteasen erzeugt, die ebenfalls einen Erfindungsgegenstand darstellen. Diese erfindungsgemäßen, stabilisierten hochalkalischen Proteasen zeichnen sich dadurch aus, daß sie eine Aminosäurensequenz besitzen, welche mindestens 80 %, vorzugsweise mindestens 90 %, insbesondere aber mindestens 95 % Homologie zu der in Fig. 1 angegebenen Aminosäurensequenz aufweist und sich von dieser in einer der Positionen 42, 57, 96, 114, 115, 166, 255 der Fig. 1 oder in einer der dazu homologen Positionen dadurch unterscheidet, daß die in der betreffenden Position befindliche Aminosäure durch eine Aminosäure mit einem neutralen voluminöseren oder einem anionischen Aminosäurerest ausgetauscht ist. Als Beispiele für erfindungsgemäße hochalkalische Proteasen seien insbesondere solche Proteasen genannt, in deren Aminosäurensequenz die in einer der Austauschpositionen ursprünglich befindliche Aminosäure z.B. wie folgt ausgetauscht ist: in Position 42, insbesondere durch Glutaminsäure, Glutamin, Methionin oder gewünschtenfalls durch Phenylalamin oder Tyrosin; in Position 57 durch Tyrosin oder Tryptophan; in Position 255 durch Glutaminsäure, Glutamin oder Asparaginsäure.

Die Erfindung umfaßt weiterhin auch die DNA-Sequenzen, die für die vorstehenden erfindungsgemäßen hochalkalischen Proteasen codieren. Diese erfindungsgemäßen DNA-Sequenzen zeichnen sich dadurch aus, daß sie für eine hochalkalische Protease codieren, deren Aminosäurensequenz mindestens 80 %, vorzugsweise mindestens 90 %, insbesondere aber mindestens 95 % Homologie zu der in Fig. 1 angegebenen Aminosäurensequenz aufweist und sich von dieser in einer der Positionen 42, 57, 96, 114, 115, 166, 255 der Fig. 1 oder in einer der dazu homologen Positionen dadurch unterscheidet, daß die in der betreffenden Position befindliche Aminosäure durch eine Aminosäure mit einem neutralen voluminöseren oder einem anionischen Aminosäurerest ausgetauscht ist. Vektoren, insbesondere Plasmide, die eine dieser erfindungsgemäßen DNA-Sequenzen enthalten, und die mit diesen Vektoren transformierten Mikroorganismen, inbesondere mit diesen Vektoren tranformierte Bacilli, gehören ebenfalls zur Erfindung.

Die erfindungsgemäß stabilisierten hochalkalischen Proteasen eignen sich hervorragend für die Anwendung in festen und flüssigen Wasch- und Reinigungsmittelformulierungen, insbesondere aber für die Anwendung in Flüssigformulierungen. Diese Wasch- und Reinigungsmittelformulierungen können in an sich üblicher Weise formuliert werden. Die erfindungsgemäß stabilisierten Proteasen können dazu z.B. in Form von Granulaten, Prills oder Pellets, gegebenenfalls auch mit Oberflächenüberzügen versehen, oder bei Flüssigformulierungen auch in gelöster Form, mit den anderen Komponenten der Wasch- und Reinigungsmittelformulierung in an sich bekannter Weise vermischt werden. Die erfindungsgemäß stabilisierten Proteasen können in den Formulierungen in an sich für Wasch- und Reinigungsmittelenzyme üblichen Mengen, insbesondere in Mengen von bis zu 3 Gew.-% (bezogen auf die Trockensubstanz der Gesamtzusammensetzung), vorzugsweise in einer Menge von 0,2 bis 1,5 Gew.-%, verwendet werden.

Erläuterungen zu den Figuren:

Figur 1:

Sequenzprotokoll für die DNA-Sequenz des AvaI/HindIII-Fragmentes mit dem Strukturgen der hochalkalischen Ausgangsprotease aus Bacillus alcalophilus HA1, sowie die Aminosäurensequenz dieser Ausgangsprotease.

Figur 2:

Restriktionskarte des Plasmides pCLEAN4.

Figur 3:

Beispiele für DNA-Sequenzen für zur gerichteten Mutagenese verwendete synthetische Oligonukleotide und Angabe eliminierter bzw. erzeugter Erkennungsstellen für einzelne Restriktionsendonukleasen; die gegenüber der ursprünglichen DNA-Sequenz der Ausgangsprotease erzeugten Nukleotidveränderungen sind durch Angabe der veränderten Nukleotide mit kleinen Buchstaben gekennzeichnet.

Figur 4:

Restriktionskarte des Vektors pCLMUTN1.

Figur 5:

Restriktionskarte des Vektors pCLMUTC1.

Figur 6:

Restriktionskarte des Vektors pUBC132.

Figur 7:

Restriktionskarte des Plasmides pAL1P.

Figur 8:

Restriktionskarte der Expressionsvektoren vom Typ pAL1NC für die Expression der durch Mutation stabilisierten hochalkalischen Proteasen und der nicht-mutierten hochalkalischen Ausgangsprotease.

Figuren 9 bis 11:

Einfluß von anionischen Tensiden auf die Aktivität von hochalkalischen Proteasen. Die proteolytischen Aktivitäten sind in Prozent der Anfangsaktivität angegeben. Fig. 9 und 11 zeigen Proteaseaktivitäten in Gegenwart von SDS (Natriumdodecylsulfonat); Fig. 10 zeigt Proteaseaktivitäten in Gegenwart von LAS (Lineare Alkylsulfonate).

## Beispiele

Die folgende Offenbarung gibt zur weiteren Erläuterung der Erfindung typische beispielhafte Ausgestaltungen der Erfindung wieder, ohne jedoch dadurch die Erfindung zu beschränken.

Um die Beispiele zu vereinfachen werden einige häufig wiederkehrende Methoden und Begriffe im folgenden näher erläutert und dann in den einzelnen Beispielen nur noch durch eine Kurzbezeichnung referiert. Sofern nicht anders angegeben, wurde generell nach Methoden gearbeitet, wie sie in Maniatis et al. (Maniatis et al. = T. Maniatis, E. F. Fritsch, J. Sambrook, Molecular Cloning, A Laboratory Manual, Cold Spring Harbor Laboratory, 1982) beschrieben sind.

Hier verwendete Ausgangsvektoren sind käuflich und auf unbeschränkter Basis verfügbar; oder sie können nach an sich bekannten Methoden aus verfügbaren Vektoren hergestellt werden. Beispielsweise wurden Phagemide mit der Bezeichnung pBS (z.B. pBS(+), pBS(-) etc.) von Stratagene, La Jolla, Kalifornien bezogen; der Helfer-Phage M13K07 von Bio-Rad Laboratories, Richmond, Kalifornien; der Vektor M13tg131 von Amersham, Buckinghamshire, England; das Plasmid pUC18 von Pharmacia LKB, Uppsala, Schweden.

Die verschiedenen benutzten Restriktionsendonukleasen gehören zum Stand der Technik und sind kommerziell verfügbar. Die bei Verwendung dieser bekannten Restriktionsendonukleasen jeweils erforderlichen Reaktions-, Kofaktor- und übrigen Bedinungen sind ebenfalls bekannt. Z.B. kann für eine Menge von etwa 1 µg eines Vektors oder eines DNA-Fragments eine Einheit (= 1 U; U = unit) der Restriktionsendonuklease in etwa 20 µl einer Pufferlösung eingesetzt werden. Ausreichende Inkubationszeiten von etwa einer Stunde bei 37 °C wurden gewöhnlich eingehalten, die Inkubationsbedingungen können aber den gegebenen Erfordernissen angepaßt werden. Nach Inkubation mit einer Restriktionsendonuklease wurde das Protein durch Extraktion (z.B. mit Phenol und Chloroform) entfernt und die geschnittene DNA (z.B. aus der wäßrigen Fraktion durch Fällung mit Ethanol) isoliert und der weiteren Verwendung zugeführt.

An das Schneiden von Vektoren mit Restriktionsendonukleasen kann sich gegebenenfalls eine Hydrolyse des terminalen 5'-Phosphatrestes mit einer alkalischen Phosphatase (Dephosphorylierung) anschließen. Dadurch kann verhindert werden, daß die beim Schneiden entstandenen Enden des restringierten Vektors mit sich selbst rekombinieren und somit die gewünschte Insertion eines Fremd-DNA-Fragmentes in die Restriktionsstelle verhindert würde. Sofern in den Beispielen eine Dephosphorylierung des 5'-Endes vorgenommen wurde, geschah dieses in an sich bekannter Weise. Weitere Angaben zur Durchführung einer Dephosphorylierung und zu dafür benötigten Reagentien können Maniatis et al. (S. 133 - 134) entnommen werden.

Partielle Hydrolyse bedeutet unvollständige Verdauung von DNA durch eine Restriktionsendonuklease. Die Reaktionsbedingungen werden dabei so gewählt, daß in einem DNA-Substrat zwar an einigen, nicht aber an allen Erkennungsstellen für die eingesetzte Restriktionsendonuklease geschnitten wird.

Zur Gewinnung und Isolierung von bestimmten DNA-Fragmenten, z.B. nach Behandlung von DNA mit Restriktionsendonukleasen, wurden die angefallenen DNA-Fragemente in an sich bekannter Weise durch Gelelektrophorese (z.B. auf Agarosegel) getrennt, nachfolgend über das Molekulargewicht (Bestimmung durch Vergleich mit Referenz-DNA-Fragmenten mit bekanntem Molekulargewicht) identifiziert und die gewünschten DNA-Fragmente aus den entsprechenden Gelzonen abgetrennt.

Behandlung mit dem Klenow-Fragment der DNA-Polymerase I aus E. coli bedeutet ein Verfahren zum Auffüllen der inneren 3'-Enden von doppelsträngiger DNA mit Nukleotiden, die zu den Nukleotiden der jeweiligen überstehenden 5'-Enden des DNA-Doppelstranges komplementär sind. Dieses Verfahren wird z.B. verwendet, wenn innere DNA-Strangenden, die aus einer Spaltung von doppelsträngiger DNA mit Restriktionsendonukleasen resultieren, mit Nukleotiden aufgefüllt werden sollen, z.B. um für weitere Ligationen erforderliche glatte DNA-Doppelstrangenden zu erzeugen. Die Behandlung mit dem Klenow-Fragment wird ausgeführt, indem man die geeigneten komplementären Nukleotide mit der aufzufüllenden DNA in Gegenwart einer ausreichenden katalytischen Aktivität des Klenow-Fragments der E. coli-DNA-Polymerase I reagieren läßt (z.B. ca. 15 Min. bei 15 °C). Das Klenow-Fragment sowie weitere für die Klenow-Behandlung benötigte Reagentien sind im Stand der Technik bekannt und kommerziell verfügbar. Weitere Details zur Klenow-Behandlung sind z.B. aus Maniatis et al. (S. 107 bis 108) entnehmbar.

Ligation (ligieren) bedeutet ein Verfahren zur Bildung von Phosphodiesterbindungen zwischen DNA-Fragmenten (siehe z.B. Maniatis et al., S. 146). Ligationen können unter an sich bekannten Bedingungen, z.B. in einem Puffer mit etwa 10 Units T4-DNA-Ligase pro 0,5 µg einer etwa gleich molaren Menge der zu ligierenden DNA-Fragmente, ausgeführt werden.

Unter Transformation wird die Einschleusung von DNA in einen Mikroorganismus verstanden, so daß die DNA in diesem repliziert bzw. exprimiert werden kann. Für die Transformation von E. coli ist z.B. die Calciumchloridmethode nach Mandel et al. (1970, J. Mol. Biol. 53: 159) oder nach Maniatis et al. (S. 250 bis 251) geeignet. Für Bacillus-Spezies ist z.B. die Methode Anagnostopulos et al. (1961, J. Bact. 81: 741 - 746) geeignet.

Ein Linker ist ein kurzkettiges doppelsträngiges DNA-Fragment, welches einige Erkennungsstellen für Restriktionsendonukleasen aufweist und sich zum Verbinden von DNA-Fragmenten eignet. Linker werden z.B. beim Rekombinieren von DNA-Fragmenten zu einem Vektor eingesetzt und können zur Einführung bestimmter Erkennungsstellen für Restriktionsendonukleasen in diesen Vektor dienen.

Eine Polyklonierungsstelle (Polylinker) ist ein kurzes bis mittleres doppelsträngiges DNA-Fragment, welches eng benachbart eine Vielzahl von Erkennungsstellen für Restriktionsendonukleasen aufweist. Eine in den Beispielen verwendete, aus dem Vektor M13tg131 entstammende, Polyklonierungsstelle besitzt z.B. eine Größe von etwa 0,07 KB (Kilo Basenpaare) und weist Erkennungsstellen für 14 verschiedene Restriktionsendonukleasen auf.

Der im Beispiel 1 eingesetzte und als Bacillus alcalophillus HA1 benannte Bacillus alcalophillus-Stamm ist bei der Deutschen Sammlung von Mikroorganismen (DSM) mit der DSM-Nummer 5466 am 28. Juli 1989 hinterlegt worden. Andere verwendete Mikroorganismen sind käuflich verfügbar, z.B. Bacillus subtilis BD 244 (Bacillus Genetic Stock Center 1 A 46) oder Bacillus subtilis BD 366 (Bacillus Genetic Stock Center 1 E 6).

## Beispiel 1

Herstellung einer genomischen DNA-Bibliothek aus B. alcalophilus und Isolierung des Gens für die hochalkalische Ausgansprotease

Aus dem Naturisolat Bacillus alcalophilus HA1 (hinterlegt bei der Deutschen Sammlung von Mikroorganismen unter der Nummer DSM 5466) wurde nach der Methode von Saito et al. (1963, Biochim.Biophys.Acta. 72:619-629) chromosomale DNA isoliert und mit der Restriktionsendonuklease Sau3A partiell hydrolisiert. Die Restriktionsfragmente wurden durch Elektrophorese auf einem Agarosegel aufgetrennt und die Fragmente mit einer Größe von 3 bis 8 Kilobasen (KB) wurden isoliert.

Die isolierten und größenselektierten DNA-Fragmente aus Bacillus alcalophilus HA1 wurden mit Vektor-DNA des Plasmids pUB 110 (Gewinnung wie in Beispiel 7 beschrieben) in vitro neukombiniert.

Hierzu wurde das Plasmid pUB110 zunächst mit der Restriktionsendonuklease BamHI restringiert und anschließend mit alkalischer Phosphatase aus Kälberdarm dephosphoryliert. Anschließend wurden 2 $\mu$g der restringierten und dephosphorylierten Vektor-DNA mit 8 $\mu$g der Bacillus alcalophilus DNA-Fragmente in einem Gesamtvolumen von 100 $\mu$l mit T4-DNA Ligase 24 h bei 16 °C inkubiert.

Mit der erhaltenen in vitro neukombinierten DNA wurden Protoplasten des Stammes Bacillus subtilis BD224 nach der von S. Chang und N. Cohen (1979, Mol. Gen. Genet. 168: 111 - 115) beschriebenen Methode transformiert. Die Transformanten wurden auf Platten mit Neomycin selektiert und anschließend auf Magermilchagar überführt. Unter 13800 untersuchten Transformanten wurde eine gefunden, die durch Proteolyse des Magermilchagars einen deutlich größeren Hof bildete. Aus diesem Klon wurde die Plasmid-DNA nach Maniatis et al. isoliert. Das in diesem Plasmid enthaltene klonierte Fragment aus der B. alcalophilus-DNA hatte eine Größe von 4,1 KB und enthielt die vollständige Information für die hochalkalische Protease aus Bacillus alcalophilus HA1.

Zur Vereinfachung des weiteren Verfahrens wurde das 4,1 KB große DNA-Fragment zunächst in der Größe reduziert. Hierzu wurden die auf dem DNA-Fragment befindlichen Erkennungsstellen für Restriktionsendonukleasen durch Schneiden des Plasmids mit verschiedenen Restriktionsendonukleasen und Auftrennung der Fragmente der restringierten DNA durch Elektrophorese auf einem Agarosegel ermittelt. Es wurde ein 2,3 KB großes, durch Schneiden mit den Restriktionsendonukleasen AvaI und HindIII erhältliches DNA-Fragment ermittelt, welches die vollständige Information für die hochalkalische Protease aufwies und welches für das weitere Verfahren verwendet wurde. Hierzu wurde das obige Plasmid mit dem 4,1 KB-Fragment mit den Restriktionsendonukleasen AvaI und HindIII restringiert. Das 2,3 KB große DNA-Fragment wurde isoliert und mit dem Vektor pUB131 (Gewinnung wie in Beispiel 7 beschrieben), der zuvor ebenfalls mit AvaI und HindIII geschnitten wurde, ligiert.

Das erhaltene Plasmid, das die Bezeichnung pCLEAN4 erhielt, wurde in den Stamm B. subtilis BD224 eingebracht. Die Transformanten waren in der Lage, die hochalkalische Protease auszuscheiden, was zeigt, daß das AvaI/HindIII-Fragment das vollständige Strukturgen für die hochalkalische Protease aus B. alcalophilus HA1 enthält. Die Restriktionskarte des Plasmids pCLEAN4 ist in Fig. 2 wiedergegeben.

Beispiel 2

Sequenzierung des Strukturgens für die hochalkalische Protease

Zur Herstellung von Einzelstrang-DNA des Proteasestrukturgens wurde das Plasmid pCLEAN4 mit den Restriktionsendonukleasen AvaI und HindIII geschnitten und das etwa 2,3 KB große AvaI/HindIII-DNA-Fragment (Proteasestrukturgen) in den Phagemiden pBS (+) oder pBS (-) eingebracht. Die Nukleotidsequenz des in den isolierten Einzelstrang-Phagemiden enthaltenen Proteasegens wurde nach der Dideoxy-Kettenterminator-Methode von Sanger et al. (1977, Proc. Natl. Acad. Sci. USA 74:5463) und der Methode der basenspezifischen chemischen Spaltung des DNA-Einzelstranges nach Maxam et al. (1980, in Methods in Enzymology, Grossmann L., Moldave K., eds., Academic Press Inc., New York und London, Vol. 65, 499) bestimmt. Die ermittelte Nukleotidsequenz und die zugeordnete Aminosäurensequenz der Protease sind in Fig. 1 wiedergegeben. Der Start für die Aminosäurensequenz der reifen hochalkalischen Protease in Position 1190 der Nukleotidsequenz wurde durch Aminosäurensequenzierung des N-terminalen Endes der hochalkalischen Protease bestimmt.

Beispiel 3

Herstellung mutierter DNA-Sequenzen durch gerichtete Mutagenese

Die gerichteten Mutationen wurden in DNA-Teilsequenzen des Proteasestrukturgens mit der von Kunkel, T.A. (1985, Proc.Natl.Acad.Sci.USA 82:488-492) beschriebenen "primer extension" Technik durchgeführt. Hierzu wurden die Plasmide pCLMUTN1 (Herstellung wie in Beispiel 4 beschrieben) und pCLMUTC1 (Herstellung wie in Beispiel 5 beschrieben), die zunächst wie nachfolgend beschrieben in ihre uracylierten, einzelträngigen Analoga umgewandelt wurden, eingesetzt. Die Ausgangsvektoren pCLMUTN1 und pCLMUTC1 enthalten nicht die gesamte DNA-Sequenz des Proteasestrukturgens aus B. alcalophilus HA1, sondern nur die N-terminale Hälfte (pCLMUTN1) oder die C-terminale Hälte (pCLMUTC1) desselben.

Diese Vektoren sind als Abkömmlinge eines Phagemiden in gewissem Umfange zur Bildung von Einzelstrang-Vektor-DNA befähigt, die unter den hier gegebenen Bedinungen aus dem zur Replikation dienenden Wirtsorganismus ausgeschleust und isoliert werden konnte.

Jeder dieser Vektoren wurde nach Maniatis et al. (S. 250 bis 251) mit Hilfe der $CaCl_2$Methode in E. coli CJ236 als Wirtsorganismus eingebracht.

Da das Bakterium E. coli CJ236 (Uracil-N-Glycosylase-Mangelmutante) bei der Replikation von Vektoren statt Thymin das Nukleotid Uracil in die DNA-Sequenz des Vektors einbaut, wurden durch Kultivierung der vorstehenden Transformanten die Uracil-haltigen Analoga des Vektors pCLMUTN1 oder pCLMUTC1 erhalten. Diese Uracil-enthaltenden Vektoren sind von den gewöhnlichen Thymin-enthaltenden Vektoren bei in vitro-Reaktionen nicht unterscheidbar. Der Uracil-Gehalt in der Vektor-DNA stört in vitro DNA-Synthesen nicht, da Uracil weder in vitro noch in vivo mutagen ist und Uracil gleichermaßen wie Thymin codiert. Uracylierte Vektoren lassen sich vorteilhaft für die nachfolgenden in vitro Reaktionen der gerichteten Mutagenese einsetzen. Nach Beendigung der Reaktionen kann der Uracil-haltige DNA-Einzelstrang, der als Matrize zur Erzeugung mutierter DNA-Stränge (Vektoren) diente, durch Behandlung mit Uracil-N-Glycosylase beseitigt werden, ohne daß es einer phänotypischen Selektion von Mutanten bedarf. Die Glycosylase-Behandlung kann sowohl mit dem isolierten Enzym als auch mit einem durch Vektor-DNA transformierten E. coli-Stamm mit Uracil-N-Glycosylase-Aktivität durchgeführt werden.

Die für die gerichtete Mutagenese als Matrize benötigte, uracylierte einzelsträngige DNA der Vektoren pCLMUTN1 und pCLMUTC1 wurde hergestellt, indem mit einem der beiden Vektoren transformierte E. coli CJ236-Bakterien kultiviert wurden, die zusätzlich mit dem Helfer-Phagen M13K07 infiziert wurden.

Der Helfer-Phage selbst ist kaum vermehrungsfähig und zeigt keine störende Interaktion mit der Vektor-DNA der Vektoren pCLMUTN1 oder pCLMUTC1. Seine Aufgabe besteht in der Synthese von Hüllproteinen für die gebildete uracylierte einzelsträngige Vektor-DNA. Umhüllte Einzelstrang-Vektor-DNA wird aus dem Wirtsorganismus E. coli CJ236 ausgeschleust und kann aus dem Kulturmedium isoliert werden. Durch die Mithilfe des Helfer-Phagen wird die qualitative und quantitative Ausbeute an (hier an uracylierter) Einzelstrang-Vektor-DNA wesentlich erhöht.

Die isolierten, uracylierten DNA-Einzelstrang-Vektoren pCLMUTN1 oder pCLMUTC1 wurden mit den nach Beispiel 6 hergestellten, synthetischen Oligonukleotiden hybridisiert, die eine Mutationsstelle enthielten und gleichzeitig als Primer für die nachfolgende Ergänzung zum vollständigen DNA-Doppelstrang mit Mutation dienten.

Die Synthese des zweiten DNA-Stranges wurde unter Zugabe von Nukleotiden mit T4-DNA-Polymerase und die nachfolgende Ligation des neugebildeten Stranges mit T4-DNA-Ligase durchgeführt (Kunkel et al. 1987, Methods in Enzymol. 154, 367 - 382). Die gebildete Doppelstrang-Vektor-DNA wurde in E. coli MC1061 transformiert und die mutierten Vektoren wurden durch Überprüfen der entsprechenden unitären Restriktionsendonukleasen-Erkennungsstellen, die mit den synthetischen Oligonukleotiden eingeführt bzw. entfernt wurden, identifiziert.

Beispiel 4

Konstruktion des Vektors pCLMUTN1

Das in Beispiel 1 hergestellte Plasmid pCLEAN4 wurde mit Aval geschnitten. Die überstehenden Enden ("sticky ends") wurden unter Zugabe der benötigten Nukleotide mit Hilfe des Klenow-Fragments der E. coli DNA-Polymerase I (Maniatis et al., S. 114) zum DNA-Doppelstrang aufgefüllt. Nach anschließender Restriktion dieser DNA mit Xbal wurde das N-terminale 1618 Basenpaar (BP) umfassende Fragment des Proteasegens isoliert und in die Smal/Xbal-Stelle von pBS kloniert. Der resultierende Vektor erhielt die Bezeichnung pCLMUTN1. Die Restriktionskarte dieses Vektors ist in Fig. 4 wiedergegeben.

Beispiel 5

Kontruktion des Vektors pCLMUTC1

Das in Beispiel 1 hergestellte Plasmid pCLEAN4 wurde mit den Restriktionsendonukleasen Xbal und Asp718 geschnitten. Das 658 BP umfassende Xbal/Asp718-Doppelstrang-DNA-Fragment, welches die C-terminale Hälfte des Proteasestrukturgens umfaßt, wurde in die Xbal/Asp718-Stelle von pBS kloniert. Der resultierende Vektor erhielt die Bezeichnung pCLMUTC1. Die Restriktionskarte des Vektors ist in Fig. 5 wiedergegeben.

Beispiel 6

Synthese künstlicher Oligonukleotide für die gerichtete Mutagenese

Synthetische Oligonukleotide wurden nach Beaucage S.L. und Caruthers M.H. (1981, Tetrahedron Letters 22: 1859 - 1862) mit $\beta$-Cyanoethyl-phosphoramidit in einem Cyclone Synthetiser (Biosearch) hergestellt. Die erhaltenen Oligonukleotide wurden gereinigt durch Elution aus Polyacrylamidgelen und anschließende Entsalzung mit Hilfe von Sephadex-G25-Säulen. Beispiele für die synthetisierten Nukleotidsequenzen und deren Eigenschaften sind in Fig. 3 wiedergegeben. Die Sequenzen der synthetischen Oligonukleotide, die im Verfahren nach Beispiel 3 zur Einführung der Mutationen in das Proteasegen dienten, waren so gewählt, daß sie die nachfolgenden Bedingungen erfüllten.

- Die DNA-Sequenz der synthetischen Oligonukleotide war zur entsprechenden Sequenz des Proteasegens noch soweit komplementär, daß ausreichende Hybridisierungsfähigkeit derselben sichergestellt war.
- Austausch eines oder mehrerer Nukleotide innerhalb des Codons, welches für die auszutauschende Aminosäure codiert, durch andere Nukleotide, so daß dieses mutierte Codon nunmehr für eine Aminosäure mit einem voluminöseren und/oder ionischen Aminosäurerest codierte (Mutationen). Für die neue Aminosäure wurde dasjenige Codon eingesetzt, welches im Proteasegen für die entsprechende Aminosäure am häufigsten vorgefunden wurde.
- Austausch von weiteren Nukleotiden innerhalb anderer Codons, so daß die ursprüngliche Codierung der Aminosäure zwar erhalten blieb, aber dadurch im Proteasegen vorkommende Erkennungssequenzen für Restriktionsendonukleasen entfernt oder neue erzeugt wurden. Diese dienten im Verfahren nach Beispiel 3 zur Erleichterung des Screenings nach den Vektoren mit den mutierten DNA-Sequenzen für die neuen hochalkalischen Proteasen.

Beispiel 7

Isolierung und Reinigung des Plasmids pUB110 und Konstruktion des Vektors pUB131

Aus dem Stamm Bacillus sübtilis BD366 wurde nach der Methode von T.J. Gryczan et al. (1978,J.Bacteriol. 134: 318-329) das Plasmid pUB110 isoliert und anschließend nach Maniatis et al. (S. 93) über Cäsiumchlorid-Dichtegradientenzentrifugation gereinigt. Der Vektor pUB110 enthält eine nur einmal vorkommende Restriktionsstelle für die Restriktionsendonuklease BamHI und als Marker eine DNA-Sequenz, die für Antibiotikaresistenz gegenüber Neomycin codiert, sowie für die Replikation in Bacillus-Spezies benötigte DNA-Sequenzen ("origin of replication")

Das vorstehend erhaltene Plasmid pUB110 wurde mit EcoRI und BamHI restringiert. Das kleinere agment (790 BP) wurde ersetzt durch einen aus 67 Basenpaaren bestehenden Polylinker, der zuvor als EcoRI/BglII-Fragment aus dem Vektor M13tg131 isoliert worden war. Der so erhaltene Vektor mit der Bezeichnung pUB131 ist somit ein Abkömmling von pUB110, bei dem das etwa 0,8 KB große EcoRI/BamHI Fragment deletiert und dafür eine Polyklonierungsstelle eingebaut wurde.

Beispiel 8

Konstruktion der Vektoren pUBC131 und pUBC132

Das Plasmid pUC18 wurde mit AatII und PvuII geschnitten. Das 1990 Basenpaar große Fragment mit dem β-Lactamase-Gen und dem E. coli "origin of replication" wurde isoliert. Die überstehenden Enden ("sticky ends") wurden unter Zugabe der benötigten Nukleotide mit Hilfe des Klenow-Fragmentes der E. coli DNA-Polymerase I (Maniatis et al., S. 114) zum DNA-Doppelstrang aufgefüllt. Das Fragment wurde anschließend in die SnaBI-Stelle des nach Beispiel 7 erhaltenen Vektors pUB131 eingebaut, wodurch der Vektor mit der Bezeichnung pUBC131 erhalten wurde.

Das 2187 BP EcoRI/BglII-Fragment des vorstehend erhaltenen Vektors pUBC131 wurde in die EcoRI/BamHI-Stelle von pBS (+) subkloniert, wobei man den Vektor mit der Bezeichnung pBSREPU erhielt. Anschließend wurde die NcoI- bzw. StyI-Erkennungsstelle, die in der DNA-Sequenz für das repU-Polypeptid im Vektor pBSREPU vorhanden ist (I. Maciag et al. 1988, Mol. Gen. Genet. 212: 232-240), durch gerichtete Mutagenese eliminiert, indem die Nukleotidsequenz CCA TGG durch die Nukleotidsequenz CCG TGG (beide Nukleotidsequenzen codieren für die Aminosäurenfolge Tryptophan-Prolin) ersetzt wurde. Die Durchführung war analog der Verfahrensweise des Beispiels 3. Hierzu wurde uracylierte einzelsträngige DNA des Vektors pBSREPU als Matrize für die gerichtete Mutation zur Eliminierung der NcoI- bzw. StyI-Erkennungsstelle hergestellt. Anschließend wurde diese Matrize analog zur im Beispiel 3 beschriebenen "primer extension"-Technik unter Verwendung des folgenden synthetischen Oligonukleotids (hergestellt und gereinigt analog zum Verfahren zur Herstellung der synthetischen Oligonukleotide des Beispiels 6)

```
                                          NcoI/StyI
                                           Pro Trp
Originäre repU-Sequenz    : AAA GTG AGA CCA TGG AGA GAA AA
Synthetische repU-Sequenz: AAA GTG AGA CCg TGG AGA GAA AA
```

zum DNA-Doppelstrang-Vektor ergänzt und durch Transformation und Kultivierung von E. coli MC1061 die nunmehr NcoI- bzw. StyI-Erkennungstellen-freien Vektoren isoliert. Das 1726 BP EcoRI/ApaI-Fragment des isolierten Vektors wurde in die EcoRI/ApaI-Stelle von pUBC131 eingeführt. Der neue Vektor, dessen Restriktionskarte in Fig. 6 wiedergegeben ist, erhielt die Bezeichnung pUBC132.

Beispiel 9

Konstruktion des Plasmids pAL1P

Das Plasmid pAL1P wurde hergestellt durch Ligation der folgenden drei Elemente:

- das 2218 Basenpaar große AvaI/NcoI-Fragment von pCLEAN4; das Fragment enthält den Promotor und die Prepro-Region der hochalkalischen Ausgangsprotease.
- der folgende synthetische Linker mit einzelnen Erkennungsstellen für die Restriktionsendonukleasen NcoI, XbaI und Asp718, die die Einführung der mutierten N-terminalen bzw. C-terminalen Hälften des Proteasegens aus den mutierten Vektoren pCLMUT1 bzw. pCLMUTC1 oder die Einführung des gesamten Gens der Ausgangsprotease aus dem Plasmid pCLEAN4 ermöglichen;

```
5' - CCATGGTCTAGAGGTACCA - 3'
     3' - CAGATCTCCATGGTTCGAA - 5'
     ^    ^    ^    ^
     NcoI XbaI Asp718
                    HindIII
```

Der vorstehende doppelsträngige synthetische Linker mit überstehenden 5'-Enden wurde hergestellt, indem man zunächst die beiden Einzelstrang-DNA-Sequenzen analog zur Synthese der synthetischen Oligonukleotide in Beispiel 6 jeweils für sich herstellte und reinigte. Die so erhaltenen Einnzelstränge wurden nachfolgend miteinander zum Doppelstrang hybridisiert.

- das 5776 Basenpaar große AvaI/HindIII-Fragment aus dem in Beispiel 8 hergestellten Vektor pUBC132; dieses Fragment enthält DNA-Sequenzen zur Replikation und selektierbare Marker in E. coli, sowie DNA-Sequenzen zur Replikation und selektierbare Marker in B. subtilis, B. licheniformis und B. alcalophilus.

Die Konstruktion des Vektors pAL1P wurde in E. coli MC1061 durchgeführt und der Vektor aus Ampicillin-resistenten E. coli-Transformanten isoliert. Die Restriktionskarte des erhaltenen Vektors ist in Fig. 7 wiedergegeben.

## Beispiel 10

Konstruktion der Plasmide pAL1N und pAL1C

Die Konstruktion der Vektoren pAL1N und pAL1C wurde in E. coli MC1061 durchgeführt, wobei die Vektoren aus Ampicillin-resistenten E. coli-Transformanten isoliert wurden.

Das Plasmid pAL1N wurde konstruiert, indem zunächst der in Beispiel 1 erhaltene Vektor pCLEAN4 mit den Restriktionsendonukleasen NcoI und XbaI geschnitten und das erhaltene NcoI/XbaI-Fragment anschließend in die NcoI/XbaI-Stelle des Vektors pAL1P (hergestellt nach Beispiel 9) kloniert wurde. Der hergestellte Vektor enthielt den N-terminalen Teil der DNA-Sequenz, die für das reife Enzym codiert, und die regulatorischen Elemente für die Transkription und Translation der hochalkalischen Protease, sowie die Signal-Sequenz und die Processing-Sequenz.

Das Plasmid pAL1C wurde konstruiert, indem zunächst der in Beispiel 1 erhaltene Vektor pCLEAN4 mit den Restriktionsendonukleasen XbaI und Asp718 geschnitten und das erhaltene XbaI/Asp718-Fragment in die XbaI/Asp718-Stelle des Vektors pAL1P (hergestellt nach Beispiel 9) kloniert wurde. Der hergestellte Vektor enthielt den C-terminalen Teil der DNA-Sequenz, die für die reife Protease codiert, und die regulatorischen Elemente für die Transkription und Translation der hochalkalischen Protease, sowie die Signal-Sequenz und die Processing-Sequenz.

## Beispiel 11

Kontruktion der Expressionsvektoren pAL1NC

Es wurden Expressionsvektoren mit Mutationen im C-terminalen Teil der Protease-DNA-Sequenz, Expressionsvektoren mit Mutationen im N-terminalen Teil der Protease-DNA-Sequenz und zu Vergleichszwecken auch Expressionsvektoren ohne Mutationen in der Protease-DNA-Sequenz hergestellt.

Die Konstruktion der drei nachfolgend aufgeführten Expressionsvektoren wurde jeweils in E. coli MC1061 durchgeführt. Aus Ampicillin-resistenten E. coli-Transformanten wurden die Expressionsvektoren isoliert. Zur Expression mutierter und nicht-mutierter Proteasegene wurden die in diesem Beispiel hergestellten und isolierten Expressionsvektoren in B. subtilits BD224 eingebracht. Die Selektion erfolgte hier nach Neomycin- oder Phleomycin-Resistenz. Die Transformanten waren zur Produktion der durch Mutation stabilisierten (Transformanten mit Vektoren aus Abschnitten A., B. dieses Beispiels) bzw. der nicht-mutierten (Transformanten mit Vektor aus Abschnitt C. dieses Beispiels) hochalkalischen Protease befähigt. Die Restriktionskarte der hergestellten Vektoren des Typs pAL1NC ist in Fig. 8 wiedergegeben.

A. Expressionsvektor mit Mutationen im N-terminalen Teil der DNA-Sequenz der Protease

Der nach Beispiel 3 durch gerichtete Mutagenese erhaltene mutierte Vektor pCLMUTN1 wurde mit den Restriktionsendonukleasen NcoI und XbaI geschnitten. Das isolierte NcoI/XbaI-Fragment (mutierter N-terminaler Teil des Proteasestrukturgens mit den Mutationen N42R, Q57R, A96R, N114R, N115R) wurde in die NcoI/XbaI-Stelle des nach Beispiel 10 erhaltenen Plasmides pAL1C kloniert. Die erhaltenen Vektoren stellten vollständige Expressionsvektoren mit geeignetem Leseraster zur Expression der durch Mutation stabilisierten Proteasen dar.

B. Expressionsvektor mit Mutationen im C-terminalen Teil der DNA-Sequenz der Protease

Der nach Beispiel 3 durch gerichtete Mutagenese erhaltene mutierte Vektor pCLMUTC1 wurde mit den Restriktionsendonukleasen XbaI und Asp718 geschnitten. Das isolierte XbaI/Asp718-Fragment (mutierter C-terminaler Teil des Proteasestrukturgens mit den Mutationen A166R, N255R) wurde in die XbaI/Asp718-Stelle des nach Beispiel 10 erhaltenen Plasmides pAL1N kloniert. Die erhaltenen Vektoren stellten vollständige Expressionsvektoren mit geeignetem Leseraster zur Expression der durch Mutation stabilisierten Proteasen dar.

C. Expressionsvektor mit der nicht-mutierten DNA-Sequenz der Ausgangsprotease

Der Expressionsvektor mit dem nicht-mutierten Ausgangsstrukturgen der Protease wurde erhalten, indem entweder das nicht-mutierte NcoI/XbaI-Fragment aus dem nach Beispiel 1 erhaltenen Plasmid pCLEAN4 in die NcoI/XbaI-Stelle von pAL1C (Beispiel 10) kloniert wurde; oder indem das XbaI/Asp718-Fragment aus dem nach Beispiel 1 erhaltenen Plasmid pCLEAN4 in die XbaI/Asp718-Stelle von pAL1N (Beispiel 10) kloniert wurde. Die so erhaltenen Vektoren waren vollständige Expressionsvektoren mit geeignetem Leseraster zur Expression der Ausgangsprotease.

## Beispiel 12

Herstellung der durch Mutation stabilisierten hochalkalischen Proteasen und zu Vergleichszwecken auch der unstabilisierten Ausgangsprotease

50 ml Vorkulturmedium (20 g Tryptone, 10 g Hefe-Extrakt, 5 g NaCl, 75 g lösliche Stärke, 10 ml Maisquellwasser pro Liter) wurden mit einer Kolonie der zu testenden Stämme (jeweils mit einem der nach Beispiel 11 hergestellten Vektoren pAL1NC transformierte B. subtilis BD224) beimpft. Die Kultur wurde für 16 h bei 37 °C und 250 Upm inkubiert. Mit 2,5 ml dieser Kultur wurden 50 ml Hauptkulturmedium (30 g Sojamehl, 90 g Kartoffelstärke, 10 g Na-Caseinat und 10 ml Maisquellwasser pro Liter) beimpft. Die Hauptkultur wurde unter den gleichen Bedingungen wie die Vorkultur inkubiert. Nach 72 h wurden die Kulturen zentrifugiert. die gebildeten Proteasen wurden aus den Kulturbeständen mit Aceton gefällt und anschließend wie folgt gereinigt: Kationenaustauscher Mono S, FPLC; Elution mit ansteigendem Gradient 20 mMol bis 200 mMol Ammoniumacetat, pH = 6.

## Beispiel 13

Im Folgenden wurden die Stabilitätseigenschaften der stabilisierten (mutierten) Proteasen im Vergleich zur nicht-stabiliserten (nicht-mutierten) Ausgangsprotease bestimmt.

Die in Beispiel 12 erhaltenen, gereinigten Proteasen wurden für jeweils 10, 20, 30 und ggf. 60 Minuten mit einer Konzentration von 1 mg/ml bei 40 °C in 40 mMol Tris/HCl (pH = 9,5) mit 0,104 Mol SDS oder 0,104 Mol LAS inkubiert. Die proteolytischen Aktivitäten wurden anschließend mit dem Substrat N-Succinyl-Ala-Ala-Pro-Phe-p-Nitroanilid (0,3 mMol in 0,1 Mol Tris/HCl, pH = 8,6) bestimmt. Die Ergebnisse in den Tabellen 1 bis 3 und den Fig. 9 bis 11 sind um die gegebenenfalls stattfindende Selbstverdauung korrigiert und zeigen ausschließlich den Einfluß anionischer Tenside auf die Aktivität der Proteasen. Die proteolytischen Aktivitäten sind angegeben in Prozent der Ausgangsaktivität.

Die verwendeten Abkürzungen bedeuten:
SDS = Natriumdodecylsulfonat; LAS = Lineare Alkylsulfonate; WT = Ausgangsprotease, nicht stabilisiert (Wildtyp); stabilisierte Proteasen mit Mutationen in den jeweiligen Positionen der Aminosäurensequenz der Fig. 1: N42R (Position 42); Q57R (Position 57), N96R (Position 96), N114R (Position 114), N115R (Position 115), N166R (Position 166), N255R (Position 255); die Aminosäuren sind durch den Einbuchstabencode bezeichnet, wobei die ursprüngliche Aminosäure der Positionsangabe vorangestellt und die zur Stabilisierung eingeführte Aminosäure der Positionsangabe nachgestellt ist.

Tabelle 1

| Einfluß von SDS auf WT, N42R, Q57R, N144R, N255R (siehe auch Fig.9) | | | | | |
|---|---|---|---|---|---|
| Inkubationszeit [min] | Restaktivität der Proteasen in % für | | | | |
| | WT | N42R | Q57R | N114R | N255R |
| 0 | 100 | 100 | 100 | 100 | 100 |
| 10 | 43 | 94 | 80 | 51 | 65 |
| 20 | 17 | 91 | 60 | 33 | 44 |
| 30 | 12 | 87 | 48 | 32 | 37 |
| 60 | - | 78 | 23 | - | 27 |

Tabelle 2

| Einfluß von LAS auf WT, N42R, Q57R, N255R (siehe auch Fig. 10) | | | | |
|---|---|---|---|---|
| Inkubationszeit [min] | Restaktivität der Proteasen in % für | | | |
| | WT | N42R | Q57R | N255R |
| 0 | 100 | 100 | 100 | 100 |
| 10 | 49 | 96 | 68 | 68 |
| 20 | 26 | 87 | 52 | 54 |
| 30 | 21 | 81 | 43 | 44 |
| 60 | 13 | 66 | 25 | 29 |

Tabelle 3

| Einfluß von SDS auf WT, A96R, N115R, A166R (siehe auch Fig. 11) | | | | |
|---|---|---|---|---|
| Inkubationszeit [min] | Restaktivität der Proteasen in % für | | | |
| | WT | A96R | N115R | A166R |
| 0 | 100 | 100 | 100 | 100 |
| 10 | 43 | 60 | 56 | 56 |
| 20 | 17 | 35 | 32 | 30 |
| 30 | 12 | 24 | 20 | 17 |

**Patentansprüche**

1. Verfahren zur Verbesserung der Stabilität von hochalkalischen Proteasen gegen destabilisierende Einwirkung von anionischen Tensiden, dadurch gekennzeichnet, daß man in einer Protease mit einer Aminosäurensequenz mit mindestens 80 % Homologie zu der in Fig. 1 angegebenen Aminosäurensequenz in einer der Positionen 42, 57, 96, 114, 115, 166, 255 der Fig. 1 die in der betreffenden Position befindliche Aminosäure durch eine Aminosäure mit einem voluminöseren und/oder ionischen Aminosäurerest austauscht.

2. Verfahren nach Anspruch 1, dadurch gekennzeichnet, daß man eine Protease mit mindestens 90 %, vorzugsweise mindestens 95 % Homologie zu der in Fig. 1 angegebenen Aminosäurensequenz einsetzt.

3. Verfahren nach Anspruch 1, dadurch gekennzeichnet, daß man die Aminosäure in einer der Positionen 42, 57, 114, 255, vorzugsweise 42, 57 austauscht.

**4.** Verfahren nach Anspruch 1, dadurch gekennzeichnet, daß die Aminosäure mit einem voluminöseren und/oder ionischen Aminosäurerest ausgewählt ist aus der Gruppe Arginin, Lysin, Glutaminsäure, Glutamin, Asparaginsäure, Methionin, Phenylalanin, Thyrosin und Tryptophan, vorzugsweise aus der Gruppe Arginin, Lysin, Glutaminsäure, Glutamin und Asparaginsäure.

**5.** Hochalkalische Protease, gekennzeichnet durch eine Aminosäurensequenz, welche mindestens 80 %, vorzugsweise mindestens 90 %, insbesondere aber mindestens 95 % Homologie zu der in Fig. 1 angegebenen Aminosäurensequenz aufweist und sich von dieser in einer der Positionen 42, 57, 96, 114, 115, 166, 255 der Fig. 1 oder in einer der dazu homologen Positionen dadurch unterscheidet, daß die in der betreffenden Position befindliche Aminosäure durch eine Aminosäure mit einem neutralen voluminöseren oder einem anionischen Aminosäurerest ausgetauscht ist.

**6.** DNA-Sequenz, codierend für eine hochalkalische Protease, deren Aminosäurensequenz mindestens 80 %, vorzugsweise mindestens 90 %, inbesondere aber mindestens 95 % Homologie zu der in Fig. 1 angegebenen Aminosäurensequenz aufweist und sich von dieser in einer der Positionen 42, 57, 96, 114, 115, 166, 255 der Fig. 1 oder in einer der dazu homologen Positionen dadurch unterscheidet, daß die in der betreffenden Position befindliche Aminosäure durch eine Aminosäure mit einem neutralen voluminöseren oder einem anionischen Aminosäurerest ausgetauscht ist.

Fig. 1

Sequenzprotokoll

| | |
|---|---|
| ART DER SEQUENZ: | Nucleotid mit entsprechendem Protein |
| SEQUENZLÄNGE: | 2278 Basenpaare |
| STRANGFORM: | Einzelstrang |
| TOPOLOGIE: | linear |
| ART DES MOLEKÜLS: | Genom-DNA |

URSPRÜNGLICHE HERKUNFT:

| | |
|---|---|
| ORGANISMUS: | Bacillus alcalophilus |
| STAMM: | HA1, DSM 5466 |

MERKMALE:

von 1190 bis 1996 BP reifes Protein

```
CTCGGGAAGC CGATTTGCTA CTGCATGTCG TCGATTATTC AAATGAACGC     50
CATCGCGAAA TGGCAAAGAC GACAAATGAA ACACTCCAGG CAATGGAAAT    100
CGATCGCCCG ATGATTTATG TTTACAACAA AATGGATCAA GTGAAAGACG    150
CGTTTCCTCA AGCGCATGGC ACGAGCTGTT TATATCAGCT AAGGCTAAAC    200
AAGGGCTTGA TTTATTAGCA CAGAAAATAG CAAGCTATGT TTTTCAAGAT    250
TTTGAAAACA TCTCGTTCAT CATTCCTTAT CGTGACGGGG AGGCGGCTGC    300
TTATTTAAAC AACCATGCCC ATGTCACACA CAGCGTGCTG AGGAGGACGG    350
CTGGCATATC GTTGCCGATT TGCATGAACG AGACTTAAAA CGGGTTGAAA    400
GCTACTGTGT TTCAAAAGAA CGATAATGAA AAAAGCCATT TGAATGCTTC    450
TTGTTCAAAT GGCTTTTTGG CGACTATGGT AGACAGATGA ACACTTGTTT    500
CGCTGTTTTA CGACAAAGAT CATCTTGCCT GTTACGCGTT TTTTAAATCC    550
GTTTTCGCAC GTTCAATTGT CGCCGAGTCG ATCCAGTCGC TGTAAGTGAG    600
AATATGTTTA GAAAGCCGCG TATTTAAGCG CAGTCTTTTT CGTTCTGTAC    650
TGGCTGGTTT GTGGACAGTT TCCATACCCA TCAACCTCCT TTTATTTGTA    700
GCTTTCCCCA CTTGAAACCG TTTTAATCAA AAACAAGTGA GAAGATTCAG    750
TTAACTTAAC GTTAATATTT GTTTCCCAAT AGGCAAATCT TTCTAACTTT    800
GATACGTTTA AACTACCAGC TTGGACAAGT TGGTATAAAA ATGAGGAGGG    850
AACCGA ATG AAG AAA CCG TTG GGG AAA ATT GTC GCA AGC        889
       Met Lys Lys Pro Leu Gly Lys Ile Val Ala Ser
        -110                -105

 ACC GCA CTA CTC ATT TCT GTT GCT TTT AGT TCA TCG ATC      928
 Thr Ala Leu Leu Ile Ser Val Ala Phe Ser Ser Ser Ile
-100                -95                 -90

 GCA TCG GCT GCT GAA GAA GCA AAA GAA AAA TAT TTA ATT      967
 Ala Ser Ala Ala Glu Glu Ala Lys Glu Lys Tyr Leu Ile
         -85                 -80                 -75
```

Fortsetzung Fig. 1

```
GGC TTT AAT GAG CAG GAA GCT GTC AGT GAG TTT GTA GAA          1006
Gly Phe Asn Glu Gln Glu Ala Val Ser Glu Phe Val Glu
                -70                 -65

CAA GTA GAG GCA AAT GAC GAG GTC GCC ATT CTC TCT GAG          1045
Gln Val Glu Ala Asn Asp Glu Val Ala Ile Leu Ser Glu
    -60                 -55                 -50

GAA GAG GAA GTC GAA ATT GAA TTG CTT CAT GAA TTT GAA          1084
Glu Glu Glu Val Glu Ile Glu Leu Leu His Glu Phe Glu
            -45                 -40

ACG ATT CCT GTT TTA TCC GTT GAG TTA AGC CCA GAA GAT          1123
Thr Ile Pro Val Leu Ser Val Glu Leu Ser Pro Glu Asp
-35                 -30                 -25

GTG GAC GCG CTT GAA CTC GAT CCA GCG ATT TCT TAT ATT          1162
Val Asp Ala Leu Glu Leu Asp Pro Ala Ile Ser Tyr Ile
        -20                 -15                 -10

GAA GAG GAT GCA GAA GTA ACG ACA ATG GCG CAA TCA GTG          1201
Glu Glu Asp Ala Glu Val Thr Thr Met Ala Gln Ser Val
                -5                  1

CCA TGG GGA ATT AGC CGT GTG CAA GCC CCA GCT GCC CAT          1240
Pro Trp Gly Ile Ser Arg Val Gln Ala Pro Ala Ala His
 5                  10                  15

AAC CGT GGA TTG ACA GGT TCT GGT GTA AAA GTT GCT GTC          1279
Asn Arg Gly Leu Thr Gly Ser Gly Val Lys Val Ala Val
        20                  25                  30

CTC GAT ACA GGT ATT TCC ACT CAT CCA GAC TTA AAT ATT          1318
Leu Asp Thr Gly Ile Ser Thr His Pro Asp Leu Asn Ile
                35                  40

CGT GGT GGC GCT AGC TTT GTA CCA GGG GAA CCA TCC ACT          1357
Arg Gly Gly Ala Ser Phe Val Pro Gly Glu Pro Ser Thr
    45                  50                  55

CAA GAT GGG AAT GGG CAT GGC ACG CAT GTG GCC GGG ACG          1396
Gln Asp Gly Asn Gly His Gly Thr His Val Ala Gly Thr
            60                  65

ATT GCT GCT TTA AAC AAT TCG ATT GGC GTT CTT GGC GTA          1435
Ile Ala Ala Leu Asn Asn Ser Ile Gly Val Leu Gly Val
 70                 75                  80

GCG CCG AGC GCG GAA CTA TAC GCT GTT AAA GTA TTA GGG          1474
Ala Pro Ser Ala Glu Leu Tyr Ala Val Lys Val Leu Gly
        85                  90                  95

GCG AGC GGT TCA GGT TCG GTC AGC TCG ATT GCC CAA GGA          1513
Ala Ser Gly Ser Gly Ser Val Ser Ser Ile Ala Gln Gly
                100                 105

TTG GAA TGG GCA GGG AAC AAT GGC ATG CAC GTT GCT AAT          1552
Leu Glu Trp Ala Gly Asn Asn Gly Met His Val Ala Asn
    110                 115                 120
```

Fortsetzung Fig. 1

```
TTG AGT TTA GGA AGC CCT TCG CCA AGT GCC ACA CTT GAG           1591
Leu Ser Leu Gly Ser Pro Ser Pro Ser Ala Thr Leu Glu
            125                 130

CAA GCT GTT AAT AGC GCG ACT TCT AGA GGC GTT CTT GTT           1630
Gln Ala Val Asn Ser Ala Thr Ser Arg Gly Val Leu Val
135                 140                 145

GTA GCG GCA TCT GGG AAT TCA GGT GCA GGC TCA ATC AGC           1669
Val Ala Ala Ser Gly Asn Ser Gly Ala Gly Ser Ile Ser
        150                 155                 160

TAT CCG GCC CGT TAT GCG AAC GCA ATG GCA GTC GGA GCT           1708
Tyr Pro Ala Arg Tyr Ala Asn Ala Met Ala Val Gly Ala
                165                 170

ACT GAC CAA AAC AAC AAC CGC GCC AGC TTT TCA CAG TAT           1747
Thr Asp Gln Asn Asn Asn Arg Ala Ser Phe Ser Gln Tyr
    175                 180                 185

GGC GCA GGG CTT GAC ATT GTC GCA CCA GGT GTA AAC GTG           1786
Gly Ala Gly Leu Asp Ile Val Ala Pro Gly Val Asn Val
            190                 195

CAG AGC ACA TAC CCA GGT TCA ACG TAT GCC AGC TTA AAC           1825
Gln Ser Thr Tyr Pro Gly Ser Thr Tyr Ala Ser Leu Asn
200                 205                 210

GGT ACA TCG ATG GCT ACT CCT CAT GTT GCA GGT GCA GCA           1864
Gly Thr Ser Met Ala Thr Pro His Val Ala Gly Ala Ala
        215                 220                 225

GCC CTT GTT AAA CAA AAG AAC CCA TCT TGG TCC AAT GTA           1903
Ala Leu Val Lys Gln Lys Asn Pro Ser Trp Ser Asn Val
                230                 235

CAA ATC CGC AAT CAT CTA AAG AAT ACG GCA ACG AGC TTA           1942
Gln Ile Arg Asn His Leu Lys Asn Thr Ala Thr Ser Leu
    240                 245                 250

GGA AGC ACG AAC TTG TAT GGA AGC GGA CTT GTC AAT GCA           1981
Gly Ser Thr Asn Leu Tyr Gly Ser Gly Leu Val Asn Ala
            255                 260

GAA GCG GCA ACA CGC TAATCAATAA AAAAAGCCTG TGCGGTTAAA      2026
Glu Ala Ala Thr Arg
265

GGGCACAGCG TTTTTTTGTG TATGAATCGA AAAAGAGAAC AGATCGCAGG    2076
TCTCAAAAAT CGAGCGTAAA GGGCTGTTTA AAGCTCTTTA CGCTCGCAGG    2126
TCTTATCGCT ATACAATGGA AAATTCACGT CTTTTGACTT TCATGGCATA    2176
TTTATTTAAG TATTCGTTTG CTTTTTCGTA CTCTCCGTTT TTCTGGTACC    2226
ATTGCGCCAG CTCAATTGCA TAGTGGACTG GTTCTTCTTT ATTATCAAGC    2276
TT                                                        2278
```

Fig. 2

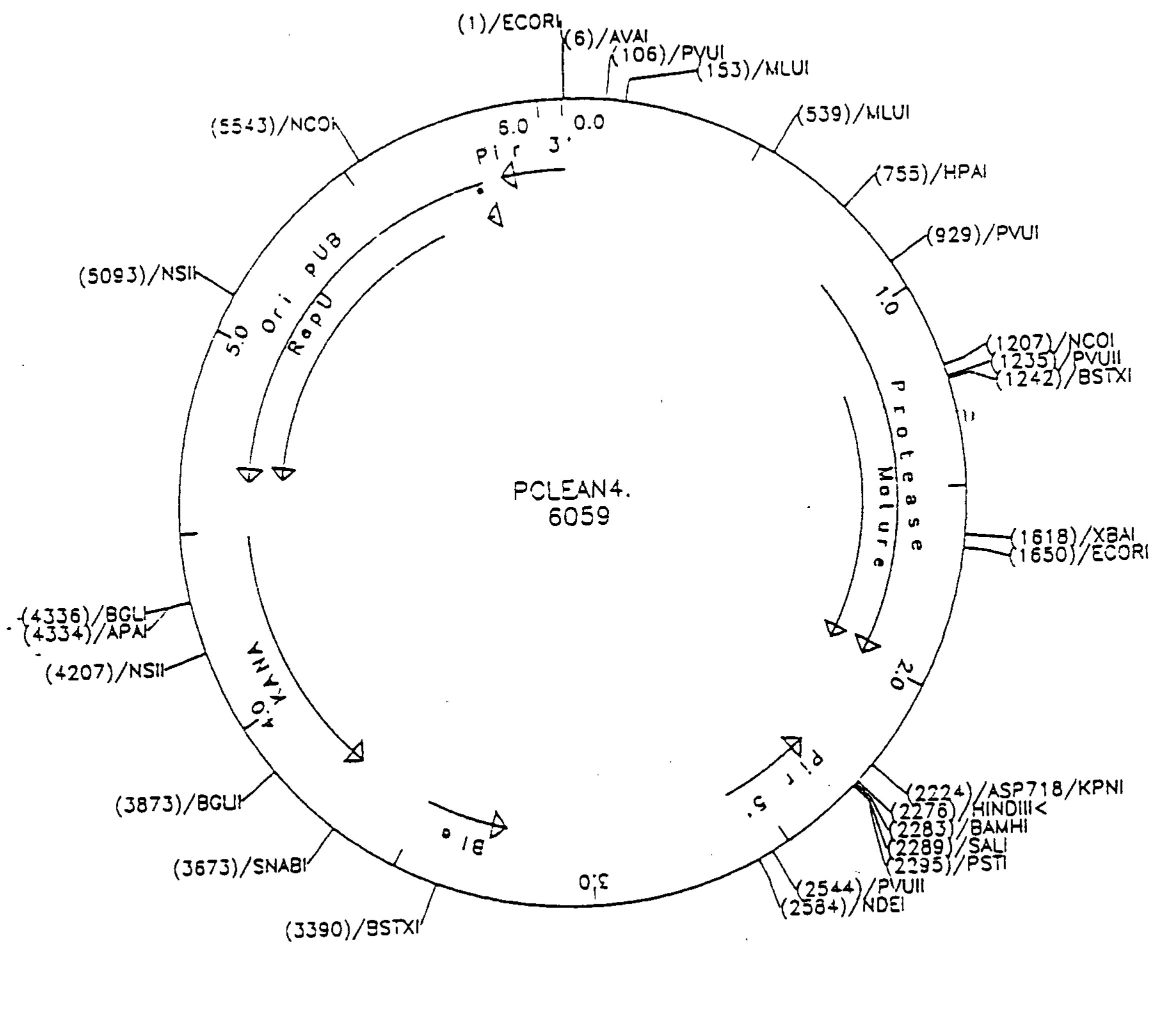
(1)/ECORI
(6)/AVAI
(106)/PVUI
(153)/MLUI
(539)/MLUI
(755)/HPAI
(929)/PVUI
(1207)/NCOI
(1235)/PVUII
(1242)/BSTXI
(1618)/XBAI
(1650)/ECORI
(2224)/ASP718/KPNI
(2276)/HINDIII<
(2283)/BAMHI
(2289)/SALI
(2295)/PSTI
(2544)/PVUII
(2584)/NDEI
(3390)/BSTXI
(3673)/SNABI
(3873)/BGUI
(4207)/NSII
(4334)/APAI
(4336)/BGLI
(5093)/NSII
(5543)/NCOI
0.0
1.0
2.0
3.0
4.0
5.0
6.0
Pir 3'
Ori pUB
RepU
Protease
Mature
Pir 5'
Ble
KANA
PCLEAN4.
6059

Fig. 3

Asn42--->Arg: (N42R)

| | | | | | | | |
|---|---|---|---|---|---|---|---|
| | 39 | 40 | 41 | 42 | 43 | 44 | 45 |
| Sequenz der Ausgangs-protease | Pro | Asp | Leu | Asn | Ile | Arg | Gly |
| : | CCA | GAC | TTA | AAT | ATT | CGT | GGT |
| | | | SnaB1 | | | | |
| Oligonucleotid : | CCA | GAC | TTA | cgt | ATT | CGT | GGT |
| Neue Aminosäure : | | | | Arg | | | |

-----------------------------------------------------------

Gln57--->Arg: (Q57R)

| | | | | | | | | | |
|---|---|---|---|---|---|---|---|---|---|
| | 53 | 54 | 55 | 56 | 57 | 58 | 59 | 60 | 61 |
| Sequenz der Ausgangs-protease | Glu | Pro | Ser | Thr | Gln | Asp | Gly | Asn | Gly |
| : | GAA | CCA | TCC | ACT | CAA | GAT | GGG | AAT | GGG |
| | | | | Mlu1 | | | | | |
| *Oligonucleotid* : | *GAA* | *CCA* | *TCC* | *ACg* | *CGt* | *GAT* | *GGG* | *AAT* | *GGG* |
| Neue Aminosäure : | | | | | Arg | | | | |

-----------------------------------------------------------

Ala96--->Arg: (A96R)

| | | | | | | | | | |
|---|---|---|---|---|---|---|---|---|---|
| | 91 | 92 | 93 | 94 | 95 | 96 | 97 | 98 | 99 |
| Sequenz der Ausgangs-protease | Val | Lys | Val | Leu | Gly | Ala | Ser | Gly | Ser |
| : | GTT | AAA | GTA | TTA | GGG | GCG | AGC | GGT | TCA |
| | | | Sca1 | | | | | | |
| Oligonucleotid : | GTT | AAA | GTA | cTt | GGG | cgt | AGC | GGT | TCA |
| Neue Aminosäure : | | | | | | Arg | | | |

-----------------------------------------------------------

Asn114--->Arg: (N114R)

| | | | | | | | | |
|---|---|---|---|---|---|---|---|---|
| | 111 | 112 | 113 | 114 | 115 | 116 | 117 | 118 |
| Sequenz der Ausgangs-protease | Trp | Ala | Gly | Asn | Asn | Gly | Met | His |
| : | TGG | GCA | GGG | AAC | AAT | GGC | ATG | CAC |
| | | | | | | | Sph1 | |
| Oligonucleotid : | TGG | GCA | GGG | cgt | AAT | GGt | ATG | CAC |
| Neue Aminosäure : | | | | Arg | | | | |

-----------------------------------------------------------

Fortsetzung Fig. 3

Asn115--->Arg: (N115R)

| | | 111 | 112 | 113 | 114 | 115 | 116 | 117 | 118 | 119 |
|---|---|---|---|---|---|---|---|---|---|---|
| Sequenz der Ausgangs-protease | : | Trp | Ala | Gly | Asn | Asn | Gly | Met | His | Val |
| | | TGG | GCA | GGG | AAC | AAT | GGC | ATG | CAC | GTT |
| | | | | BstE2 | | | | | | |
| Oligonucleotid | : | TGG | GCA | GGt | AAC | cgT | GGC | ATG | CAC | GTT |
| Neue Aminosäure | : | | | | | Arg | | | | |

---

Ala166--->Arg: (A166R)

| | | 162 | 163 | 164 | 165 | 166 | 167 | 168 | 169 | 170 |
|---|---|---|---|---|---|---|---|---|---|---|
| Sequenz der Ausgangs-protease | : | Pro | Ala | Arg | Tyr | Ala | Asn | Ala | Met | Ala |
| | | CCG | GCC | CGT | TAT | GCG | AAC | GCA | ATG | GCA |
| | | | BssH2 | | | | | Nco1 | | |
| Oligonucleotid | : | CCG | GCg | CGc | TAT | cgt | AAC | GCc | ATG | GCA |
| Neue Aminosäure | : | | | | | Arg | | | | |

---

Asn255--->Arg: (N255R)

| | | 251 | 252 | 253 | 254 | 255 | 256 | 257 | 258 | 259 |
|---|---|---|---|---|---|---|---|---|---|---|
| Sequenz der Ausgangs-protease | : | Leu | Gly | Ser | Thr | Asn | Leu | Tyr | Gly | Ser |
| | | TTA | GGA | AGC | ACG | AAC | TTG | TAT | GGA | AGC |
| | | | | | Mlu1 | | | | | |
| Oligonucleotid | : | TTA | GGA | AGC | ACG | cgt | TTG | TAT | GGA | AGC |
| Neue Aminosäure | : | | | | | Arg | | | | |

---

Fig. 4

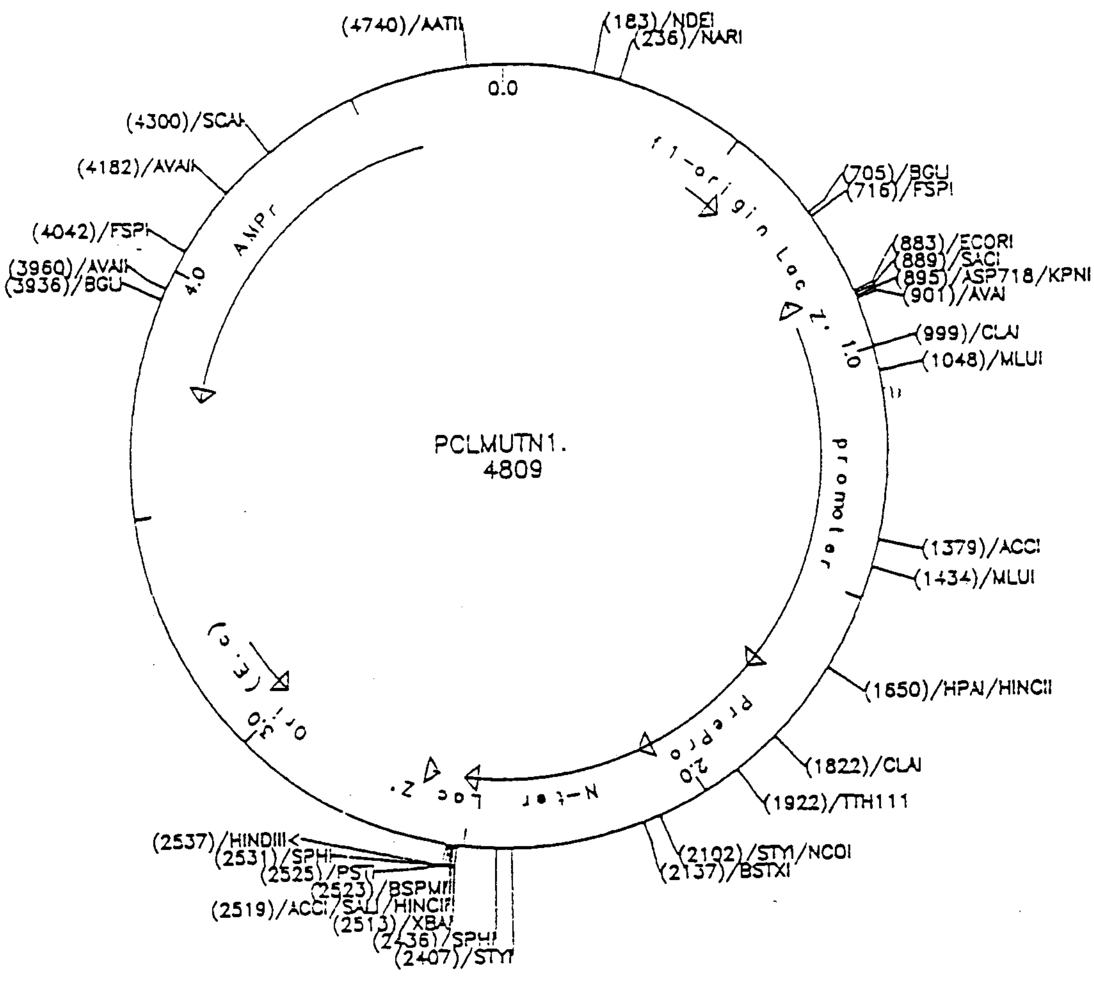
(4740)/AATII
(183)/NDEI
(236)/NARI
0.0
(4300)/SCAI
(4182)/AVAII
(4042)/FSPI
(3960)/AVAII
(3936)/BGLI
(705)/BGLI
(716)/FSPI
(883)/ECORI
(889)/SACI
(895)/ASP718/KPNI
(901)/AVAI
(999)/CLAI
(1048)/MLUI
f1-origin
Lac Z'
1.0
AMPr
4.0
PCLMUTN1.
4809
promoter
(1379)/ACCI
(1434)/MLUI
(1650)/HPAI/HINCII
ori (E.c)
3.0
Prepro
2.0
N-ter
Lac Z'
(1822)/CLAI
(1922)/TTH111
(2102)/STYI/NCOI
(2137)/BSTXI
(2537)/HINDIII
(2531)/SPHI
(2525)/PSTI
(2523)/BSPMII
(2519)/ACCI/SALI/HINCII
(2513)/XBAI
(2436)/SPHI
(2407)/STYI

Fig. 5

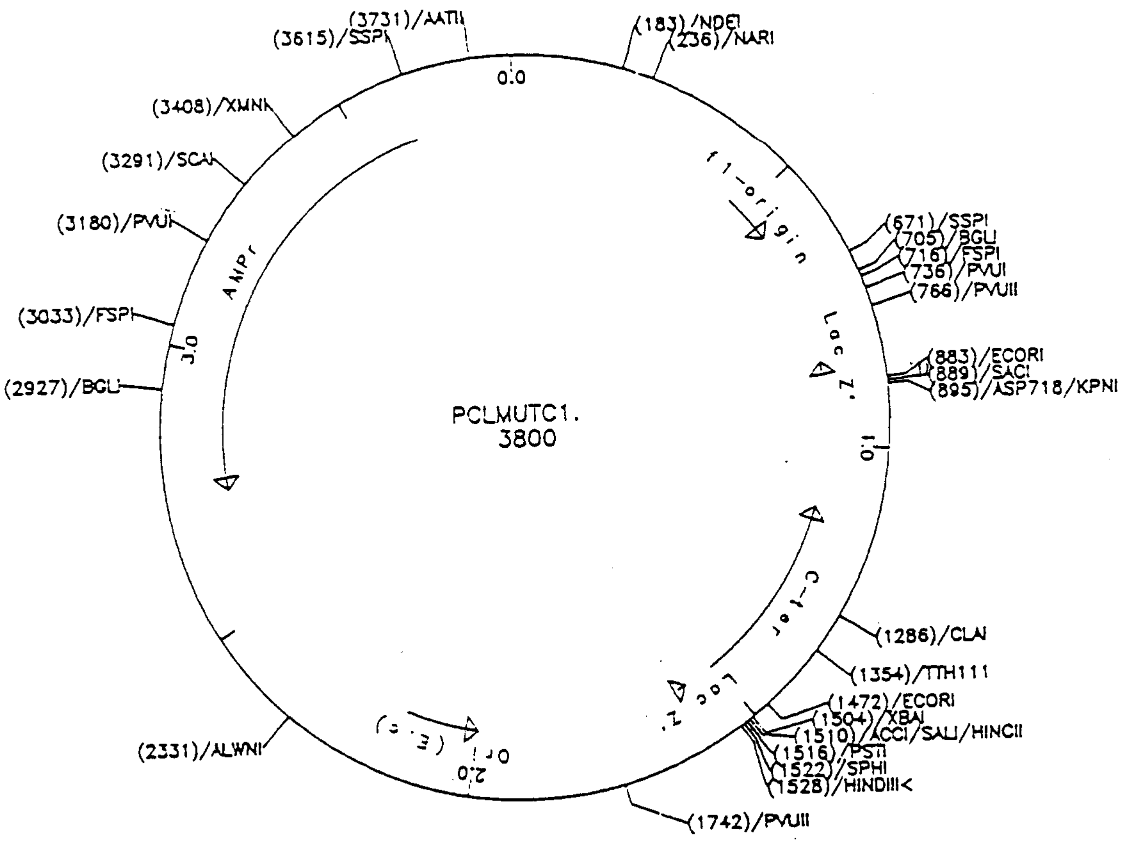
PCLMUTC1.
3800
(3731)/AATII
(3615)/SSPI
(3408)/XMNI
(3291)/SCAI
(3180)/PVUI
(3033)/FSPI
(2927)/BGLI
(2331)/ALWNI
(183)/NDEI
(236)/NARI
(671)/SSPI
(705)/BGLI
(716)/FSPI
(736)/PVUI
(766)/PVUII
(883)/ECORI
(889)/SACI
(895)/ASP718/KPNI
(1286)/CLAI
(1354)/TTH111
(1472)/ECORI
(1504)/XBAI
(1510)/ACCI/SALI/HINCII
(1516)/PSTI
(1522)/SPHI
(1528)/HINDIII<
(1742)/PVUII
0.0
1.0
2.0
3.0
f1-origin
Lac Z'
C-ter
Lac Z'
Ori (E.c)
AMPr

Fig. 6

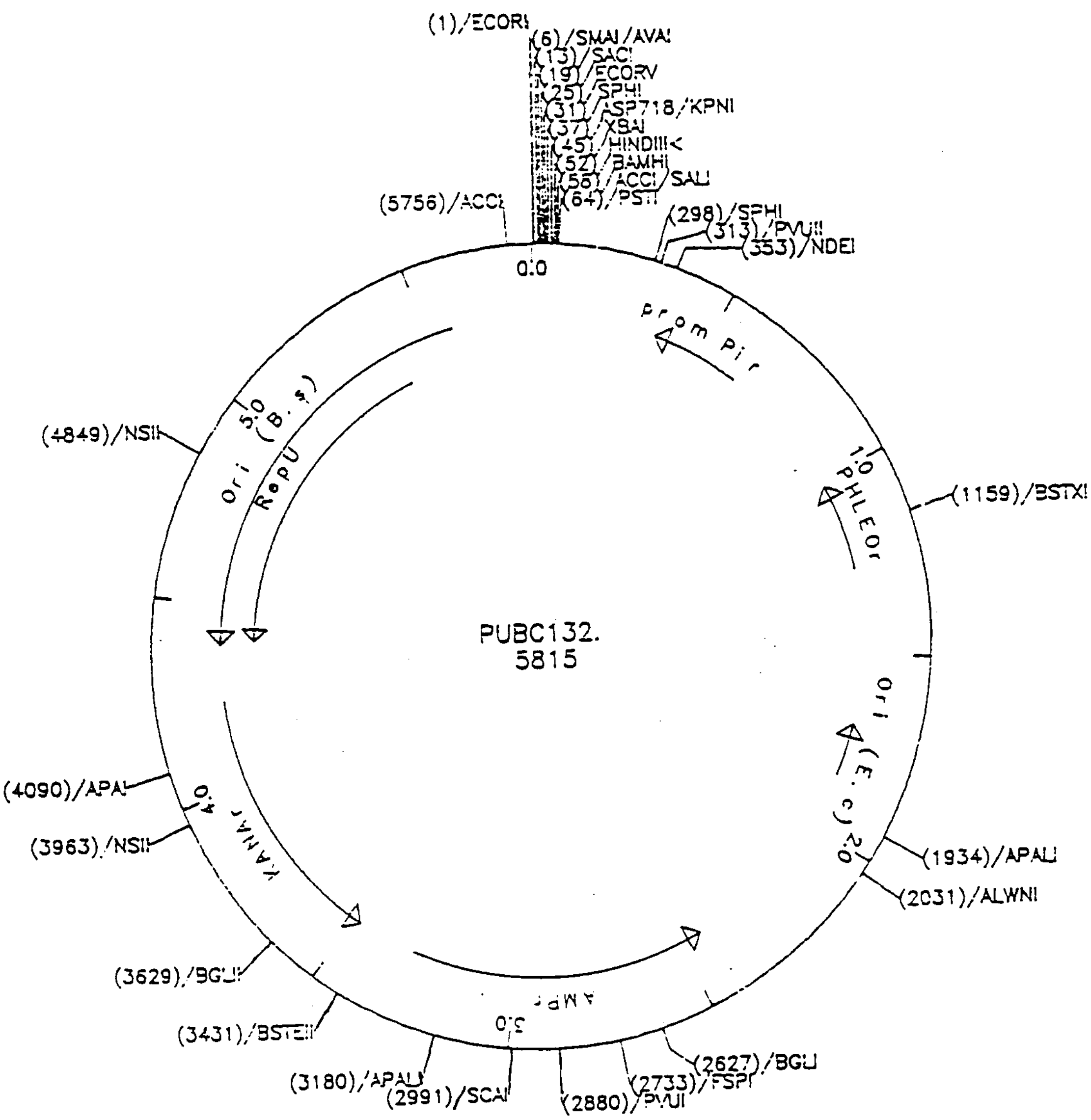
(1)/ECORI
(6)/SMAI/AVAI
(13)/SACI
(19)/ECORV
(25)/SPHI
(31)/ASP718/KPNI
(37)/XBAI
(45)/HINDIII<
(52)/BAMHI
(56)/ACCI/SALI
(64)/PSTI
(5756)/ACCI
(298)/SPHI
(313)/PVUII
(353)/NDEI
0.0
prom Pir
(4849)/NSII
5.0
Ori (B.s)
RepU
1.0
PHLEOr
(1159)/BSTXI
PUBC132.
5815
Ori (E.c)
2.0
(1934)/APALI
(2031)/ALWNI
(4090)/APAI
4.0
KANAr
(3963)/NSII
(3629)/BGLII
AMPr
3.0
(3431)/BSTEII
(3180)/APALI
(2991)/SCAI
(2880)/PVUI
(2733)/FSPI
(2627)/BGLI

Fig. 7

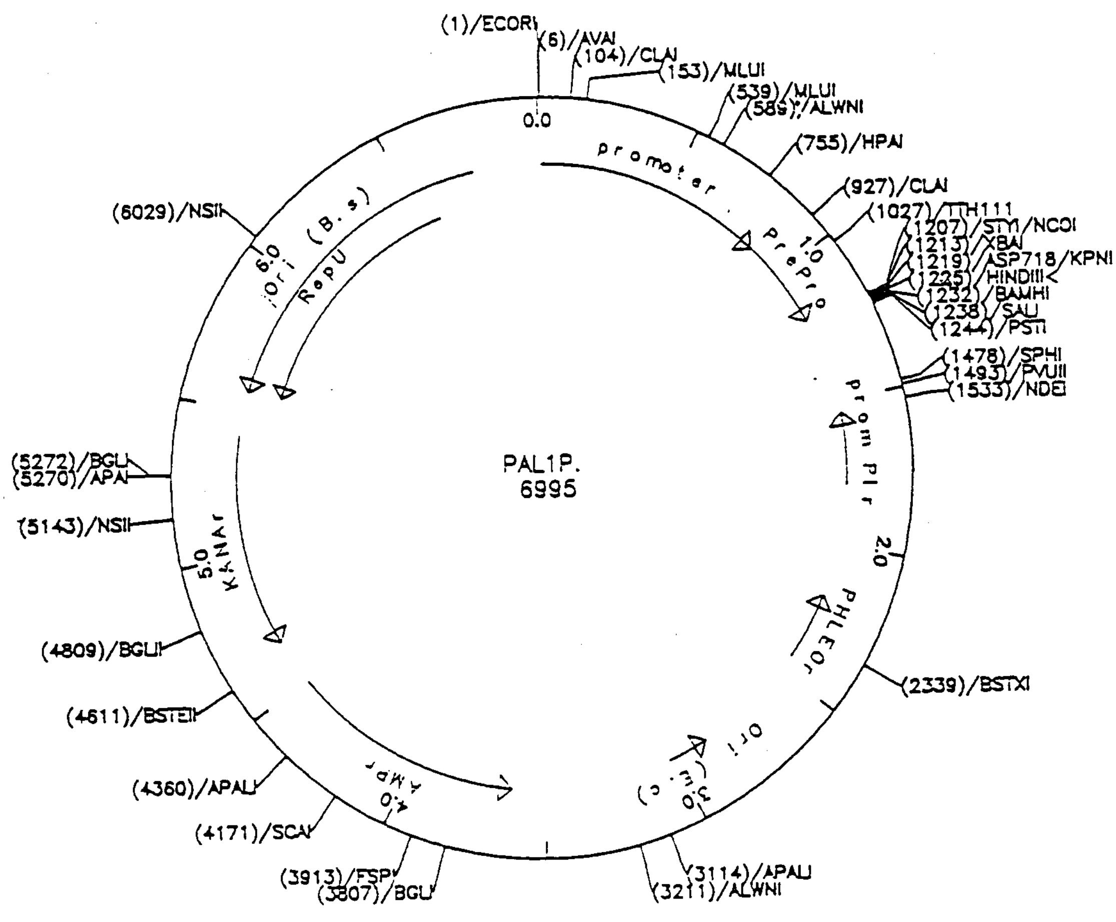
(1)/ECORI
(6)/AVAI
(104)/CLAI
(153)/MLUI
(539)/MLUI
(585)/ALWNI
(755)/HPAI
(927)/CLAI
(1027)/TTH111
(1207)/STYI/NCOI
(1213)/XBAI
(1219)/ASP718/KPNI
(1225)/HINDIII
(1232)/BAMHI
(1238)/SALI
(1244)/PSTI
(1478)/SPHI
(1493)/PVUII
(1533)/NDEI
(2339)/BSTXI
(3114)/APALI
(3211)/ALWNI
(3913)/FSPI
(3807)/BGLI
(4171)/SCAI
(4360)/APALI
(4611)/BSTEII
(4809)/BGLI
(5143)/NSII
(5270)/APAI
(5272)/BGLI
(6029)/NSII
0.0
1.0
2.0
3.0
4.0
5.0
6.0
promoter
PrePro
prom Plr
PHLEOr
ori (m.c)
AMPr
KANAr
Ori (B.s)
RepU
PAL1P.
6995

Fig. 8

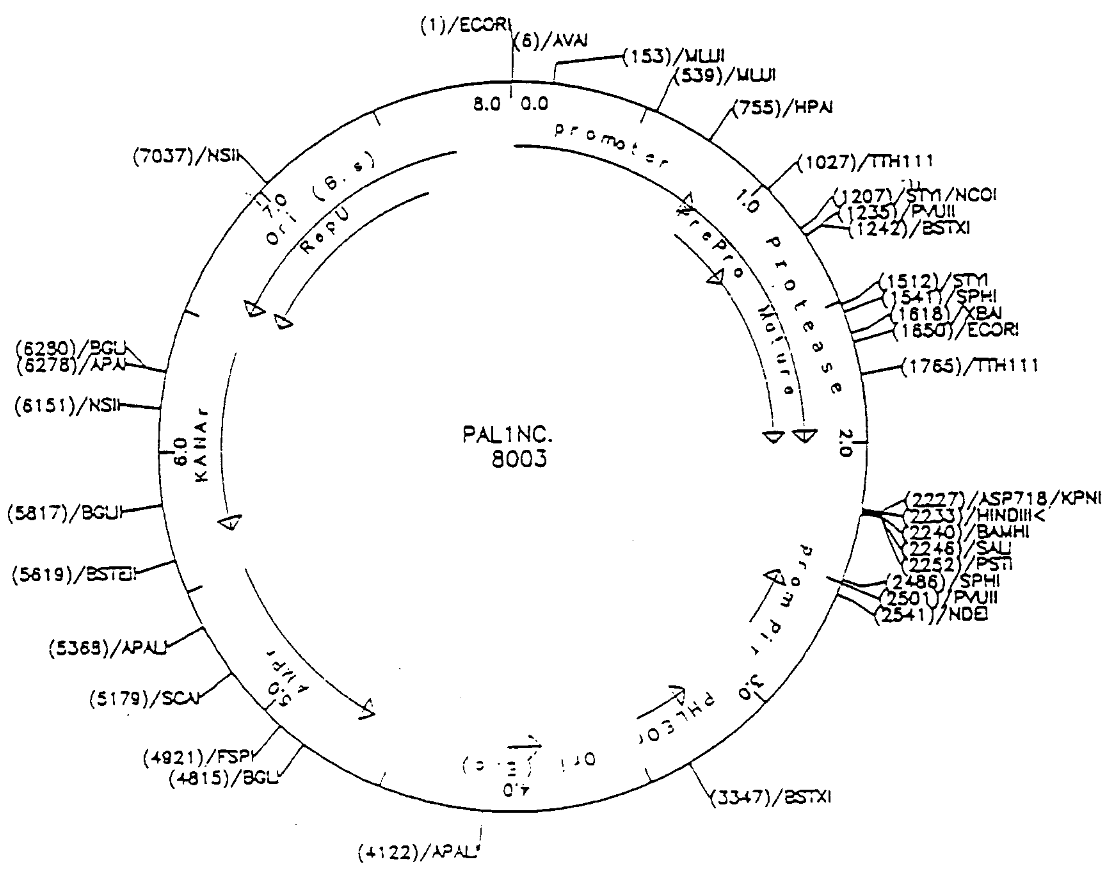
(1)/ECORI
(6)/AVAI
(153)/MLUI
(539)/MLUI
(755)/HPAI
(1027)/TTH111
(1207)/STYI/NCOI
(1235)/PVUII
(1242)/BSTXI
(1512)/STYI
(1541)/SPHI
(1618)/XBAI
(1650)/ECORI
(1765)/TTH111
(2227)/ASP718/KPNI
(2233)/HINDIII
(2240)/BAMHI
(2246)/SALI
(2252)/PSTI
(2486)/SPHI
(2501)/PVUII
(2541)/NDEI
(3347)/BSTXI
(4122)/APALI
(4815)/BGLI
(4921)/FSPI
(5179)/SCAI
(5368)/APALI
(5619)/BSTEII
(5817)/BGLI
(6151)/NSII
(6278)/APAI
(6280)/BGLI
(7037)/NSII
PAL1NC.
8003
promoter
Protease
prePro
Mature
prom pir
PHLEO
Ori
KANAr
RepU
Ori
8.0
0.0
1.0
2.0
3.0
4.0
5.0
6.0
7.0

Fig. 9

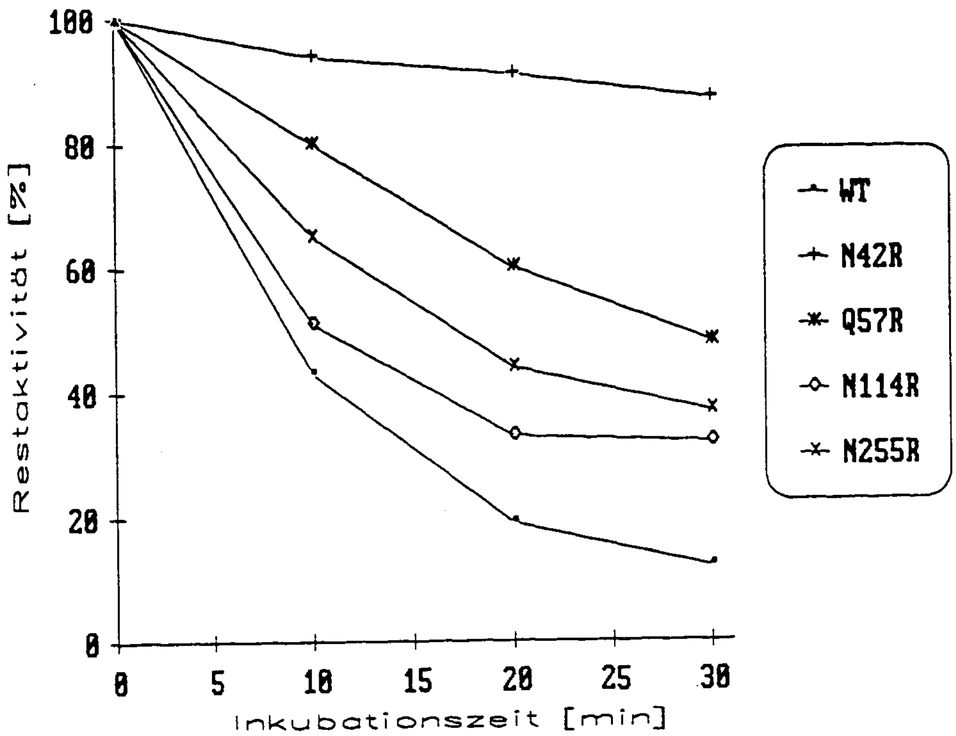
100
80
60
40
20
0
Restaktivität [%]
0
5
10
15
20
25
30
Inkubationszeit [min]
WT
N42R
Q57R
N114R
N255R

Fig. 10

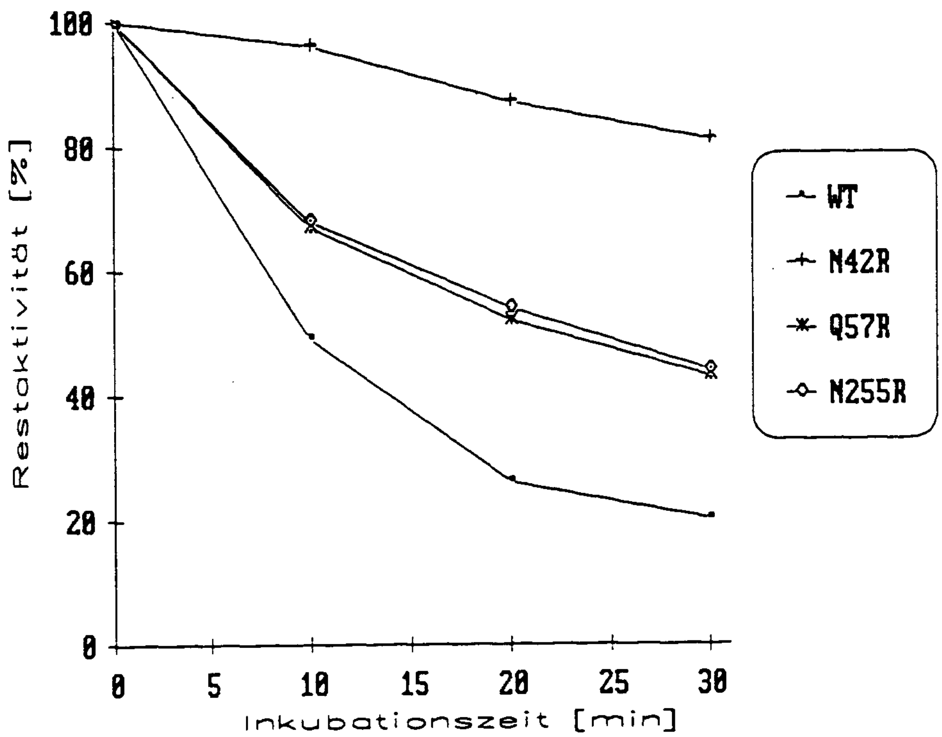
100
80
60
40
20
0
Restaktivität [%]
0
5
10
15
20
25
30
Inkubationszeit [min]
WT
N42R
Q57R
N255R

Fig. 11

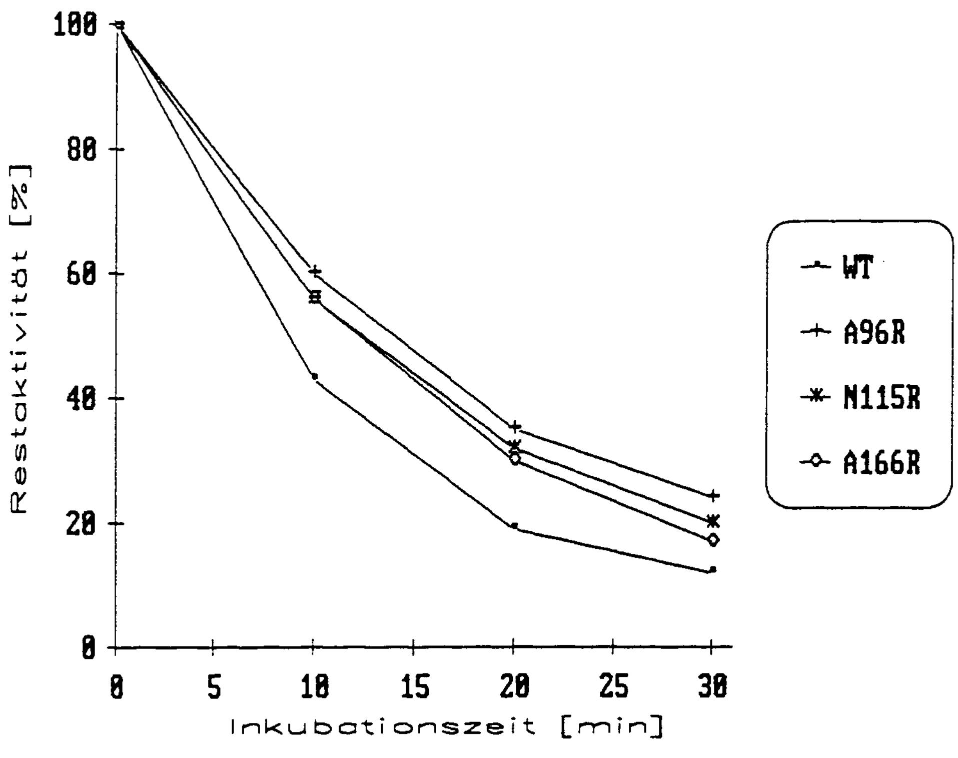
100
80
60
40
20
0
Restaktivität [%]
0
5
10
15
20
25
30
Inkubationszeit [min]
WT
A96R
N115R
A166R